# EUROPÄISCHE PATENTSCHRIFT

(11) **EP 1 781 587 B1**
(45) Veröffentlichungstag und Bekanntmachung des Hinweises auf die Patenterteilung: **02.03.2011**
(21) Anmeldenummer: 05777791.4
(22) Anmeldetag: 23.08.2005
(51) Int. Cl.: C07C 41/06, C07C 29/36, C07C 209/60, C07C 67/04

(54) **VERFAHREN ZUR TELOMERISATION VON NICHT CYCLISCHEN OLEFINEN**
METHOD FOR THE TELOMERIZATION OF NON-CYCLIC OLEFINS
PROCEDE DE TELOMERISATION D'OL FINES NON CYCLIQUES

(30) Priorität: 28.08.2004 DE 102004041778; 01.08.2005 DE 102005036038
(43) Veröffentlichungstag der Anmeldung: 09.05.2007
(73) Patentinhaber: Evonik Oxeno GmbH, 45772 Marl (DE)
(72) Erfinder: BORGMANN, Cornelia, 60486 Frankfurt (DE); RÖTTGER, Dirk, 45657 Recklinghausen (DE); ORTMANN, Dagmara, 3902 Brig-Glis (CH); BUKOHL, Reiner, 45770 Marl (DE); HOUBRECHTS, Stephan, 46286 Dorsten (DE); NIERLICH, Franz, 45768 Marl (DE)
(86) Internationale Anmeldenummer: PCT/EP2005/054136
(87) Internationale Veröffentlichungsnummer: WO 2006/024615

(56) Entgegenhaltungen:
- WO-A-91/09822
- DE-A- 10 149 348

## Beschreibung

Die Erfindung betrifft ein Verfahren zur Telomerisation von nicht cyclischen Olefinen mit mindestens zwei konjugierten Doppelbindungen, insbesondere die Herstellung von 1-Octa-2,7-dienylderivaten, durch Umsetzung eines 1,3-Butadien-haltigen Kohlenwasserstoff-gemischs, insbesondere Crack-C₄, mit Nucleophilen.

Die dabei aus zwei Molen 1,3-Butadien und einem Mol Nucleophil entstehenden Telomerisationsprodukte (ungesättigte Amine, ungesättigte Alkohole und deren Ester und Ether) sind Ausgangsstoffe für organische Synthesen. Die sauerstoffhaltigen Derivate sind Vorstufen für die Herstellung von linearen C₈-Alkoholen und C₈-Olefinen, insbesondere 1-Octanol und 1-Octen. 1-Octanol wiederum wird beispielsweise zur Erzeugung von Weichmachern verwendet 1-Octen ist ein begehrtes Comonomer zur Modifizierung von Polyethylen und Polypropylen.

Die Telomerisation von Butadien mit einem Nucleophil zu Octadienylderivaten wird durch Metallkomplexe, insbesondere Palladiumverbindungen, katalysiert.

Beispiele für Telomerisationsreaktionen werden unter anderem beschrieben in E. J. Smutny, J. Am. Chem. Soc. 1967, 89, 6793; S. Takahashi, T. Shibano, N. Hagihara, Tetrahedron Lett. 1967, 2451; EP-A-0 561 779, US 3 499 042, US 3 530 187, GB 1 178 812, NL 6 816 008, GB 1 248 593, US 3 670 029, US 3 670 032, US 3 769 352, US 3 887 627, GB 1 354 507, DE 20 40 708, US 4 142 060, US 4 146 738, US 4 196 135, GB 1 535 718, US 4 104 471, DE 21 61 750 und EP-A-0 218 100.

Als Einsatzstoffe für die Herstellung von Octadienylderivaten können reines 1,3-Butadien oder 1,3-Butadien-haltige Kohlenwasserstoffgemische, wie beispielsweise Crack-C₄, verwendet werden.

1,3-Butadien ist wegen des aufwändigen Abtrennverfahren ein relativ teurer Einsatzstoff. Daher ist es meistens wirtschaftlicher, 1,3-Butadien-haltige Kohlenwasserstoffgemische als Einsatzstoff für die Telomerisation zu wählen. Dies ist möglich, da die meisten Begleitstoffe, wie gesättigte Kohlenwasserstoffe, wie beispielsweise n-Butan oder Isobutan, oder Monoolefine, wie beispielsweise Isobuten und lineare Butene, sich in der Telomerisationsreaktion inert verhalten. Nur Inhibitoren, also Stoffe, die die Raum-Zeit-Ausbeute oder die Selektivität mindern oder den Katalysatorverbrauch erhöhen, sollten vorher abgetrennt werden.

Nach DE 195 23 335 ist es ratsam, bei Einsatz der C₄-Fraktion aus Naphthacrackern als 1,3-Butadien-haltigen Rohstoff die Konzentration von acetylenischen Verbindungen und von Allenen im Edukt für die Telomerisation zu begrenzen. Die Summe an acetylenisch und allenisch ungesättigten Verbindungen soll 1 Massen-% bezogen auf 1,3-Butadien nicht überschreiten. Für die Entfernung dieser Störkomponenten wird auf bekannte Verfahren verwiesen, ohne dass bestimmte Verfahren angeführt oder zitiert werden.

Mit Berufung auf diese Patentschrift (DE 195 23 335) weisen DE 101 49 348, DE 102 29 290 und DE 103 29 042 ohne Angabe von Konzentrationsgrenzen darauf hin, dass es günstig sei, acetylenische und allenische Verbindungen vor der Telomerisation zu entfernen.

In WO 91-09822 wird beschrieben, dass es zwecksmäßig ist, aus der beim Crackprozess von Naphtha, Gasöl oder LPG erhaltenen C₄-Mischung vor der Telomerisation acetylenischungesättigte Verbindungen durch Selektivhydrierung zu entfernen, soweit diese vorhanden sind. Das dabei verwendete Hydrierverfahren ist dabei nicht offengelegt. In den Beispielen wird ein Rohstoff mit unter 60 ppm Gesamtgehalt an Acetylenen eingesetzt, der keinen ausgewiesenen Anteil an Allenen enthält.

Die Abtrennung der acetylenischen Verbindungen kann durch Extraktion oder Hydrierung dieser Verbindungen erfolgen. Bei der Entfernung der acetylenischen Verbindungen (Methylacetylen (Propin), Ethylacetylen (Butin), Vinylacetylen (Butenin)) durch Hydrierung werden Verfahren angewandt, bei denen mit hoher Selektivität die acetylenischen Verbindungen im Wesentlichen ohne Hydrierung von 1,3-Butadien und Monoolefinen hydriert werden. Als Katalysatoren werden Hydrierkontakte verwendet, die Kupfer, Kupfer in Kombination mit unedlen Metallen, Kupfer in Kombination mit Edelmetallen oder Metallkatalysatoren von Metallen der VIII. Nebengruppe des Periodensystems der Elemente, beispielsweise Palladium-Kontakte, aufweisen. Entsprechende Verfahren sind unter anderem in folgenden Patentschriften beschrieben: US 6 576 588, US 6 417 419, US 6 225 515, US 6 015 933, US 6 194 626, US 6 040 489, US 4 493 906, US 4 440 956, US 4 101 451, US 3 912 789, US 3 751 508, US 3 541 178, US 3 327 013, US 3 218 268, EP 1 217 060, EP 1 151 790, EP1 070 695, EP 0 273 900, NL 6 613 942.

Die Entfernung von Allenen, insbesondere von 1,2-Butadien, durch Hydrierung ist wesentlich schwieriger als die selektive Entfernung von acetylenischen Verbindungen. Die Reaktivität des 1,2-Butadiens bei der Hydrierung ist nur geringfügig höher als die des 1,3-Butadiens. Daher sind bei der Entfernung von 1,2-Butadien aus 1,3-Butadien-haltigen Kohlenwasserstoffgemischen durch Hydrierung 1,3-Butadienverluste unvermeidbar.

Beispielsweise wird in WO 98/12160 ein Verfahren zur gleichzeitigen Entfernung von acetylenischen Verbindungen und 1,2-Butadien aus einem 1,3-Butadien-haltigen Kohlenwasserstoffstrom durch Hydrierung an einem Palladiumkontakt in einer Reaktivdestillations-kolonne dargelegt. Obwohl in dem dort angegebenen Beispiel 1 im Kopfprodukt der Gehalt an acetylenischen Verbindungen nur um ca. 60 % und der an 1,2-Butadiens nur um 32 % reduziert wurden, waren bereits 3 % des 1,3-Butadiens durch Hydrierung verloren.

Jeroen W. Sprengers et al. berichten in Angew. Chem. 2005, 117, 2062-2065, dass Pd-Komplexkatalysatoren, die als Liganden N-heterocyclische Liganden aufweisen, als Katalysatoren für die Hydrierung, insbesondere für die Hydrierung von 1-Phenyl-1-propin zu 1-Phenyl-1-propen und 1-Phenyl-1-propan geeignet sind.

Bei der Herstellung von 2,7-Octadienylderivaten aus Crack-C₄ durch Telomerisation gemäß dem Stand der Technik sind aufwändige Verfahren, insbesondere bezüglich des Apparateaufwands notwendig, um Inhibitoren wie Alkine aus dem Einsatzstoffgemisch abzutrennen. Diese Verfahren haben den Nachteil, dass ein Teil des 1,3-Butadiens, insbesondere wenn versucht wird den Alkin-Gehalt im Einsatzstoffgemisch unter die Nachweisgrenze zu bekommen, bei der Entfernung der Inhibitoren verloren geht. Wird zur Vermeidung von 1,3-Butadien-Verlusten auf eine weitgehend vollständige Abtrennung der Inhibitoren verzichtet, so muss eine geringere Raum-Zeit-Ausbeute oder Selektivität bei der Telomerisation oder ein höherer Katalysatorverbrauch in Kauf genommen werden.

DE10149348 beschreibt ein Verfahren zur Herstellung von 1-Olefinen unter Verwendung von Palladiumcarbenverbindungen. Dabei werden Olefine mit konjugierten Doppelbindungen mit einem Telogen in Gegenwart eines Edelmetallkatalysators telomerisiert und anschließend hydriert und zum 1-Olefin gespalten.

Aufgabe der vorliegenden Erfindung war die Bereitstellung eines alternativen Telomerisationsverfahrens welches vorzugsweise einzelne oder alle der genannten Nachteile des Standes der Technik vermeidet.

Überraschenderweise wurde nun gefunden, dass durch Zugabe von Wasserstoff über eine Wasserstoffquelle in zumindest einen Teilschritt des Gesamtprozesses der Telomerisation eine Inhibierung des Telomerisationskatalysators vermindert werden kann bzw. bei einer Inhibierung des Katalysators dieser wieder reaktiviert werden kann.

Gegenstand der Erfindung ist demnach ein Verfahren zur Telomerisation von nicht cyclischen Olefinen mit mindestens zwei konjugierten Doppelbindungen (VI) mit mindestens einem Nucleophil (VII) unter Verwendung eines Katalysators enthaltend ein Metall der Gruppe 8 bis 10 des Periodensystems der Elemente, welches dadurch gekennzeichnet ist, dass im Gesamtprozess der Telomerisation zumindest ein Teilschritt vorhanden ist, bei dem über eine Wasserstoffquelle, ausgewählt aus einem Wasserstoff aufweisendem Gas, dem in diesem Teilschritt vorliegenden Prozessgemisch Wasserstoff zugegeben wird.

Das erfindungsgemäße Verfahren hat den Vorteil, dass auf eine sehr aufwändige Abtrennung aller Inhibitoren, insbesondere aller Alkine aus dem Einsatzstoffgemisch verzichtet werden kann. Neben dem verringerten apparativen Aufwand hat das Verfahren außerdem den Vorteil, dass auch der Einsatz von kostenintensivem Hydrierkatalysator vermieden werden kann.

Das erfindungsgemäße Verfahren hat zudem den Vorteil, dass Telomerisationskatalysator eingespart werden kann, da der Telomerisationskatalysator, wenn überhaupt, deutlich langsamer deaktiviert wird.

Das erfindungsgemäße Verfahren hat außerdem den Vorteil, dass die Allene bzw. Kumulene, also die kumulierte Doppelbindungen aufweisenden Verbindungen, wie 1,2-Butadien, die einen wichtigen Ausgangsstoff für die organische Synthese darstellen, weitgehend nicht zerstört werden, sondern im Kohlenwasserstoffstrom vorhanden bleiben und nach dem zweiten Verfahrensschritt, der Telomerisation, bei der Aufarbeitung des Telomerisationsproduktes abgetrennt werden können.

Nachfolgend wird das erfindungsgemäße Verfahren beispielhaft beschrieben, ohne dass die Erfindung, deren Schutzbereich sich aus den Ansprüchen und der Beschreibung ergibt, darauf beschränkt sein soll. Auch die Ansprüche selbst gehören zum Offenbarungsgehalt der vorliegenden Erfindung. Sind nachfolgend Bereiche, allgemeine Formeln oder Verbindungsklassen angegeben, so sollen diese nicht nur die entsprechenden Bereiche oder Gruppen von Verbindungen erfassen, die explizit erwähnt sind, sondern auch alle Teilbereiche und Teilgruppen von Verbindungen, die durch Weglassen von einzelnen Werten (Bereichen) oder Verbindungen erhalten werden können.

Das erfindungsgemäße Verfahren zur Telomerisation von nicht cyclischen Olefinen mit mindestens zwei konjugierten Doppelbindungen (VI) mit mindestens einem Nucleophil (VII) unter Verwendung eines Katalysators enthaltend ein Metall der Gruppe 8, 9 oder 10 des Periodensystems der Elemente, zeichnet sich dadurch aus, dass im Gesamtprozess der Telomerisation zumindest ein Teilschritt vorhanden ist, bei dem über eine Wasserstoffquelle, ausgewählt aus einem Wasserstoff aufweisendem Gas, dem in diesem Teilschritt vorliegenden Prozessgemisch Wasserstoff zugegeben wird. Das Wasserstoff aufweisende Gas kann in situ z. B. aus Hydrazin gewonnen werden.

Als Wasserstoffquelle kann insbesondere ein Wasserstoff aufweisendes Gas, vorzugsweise Wasserstoffgas allein oder im Gemisch mit für die Telomerisation inerten Gasen, wie beispielsweise Stickstoff, Methan oder Edelgas(e), eingesetzt werden.

Vorzugsweise erfolgt die Zugabe der Wasserstoffquelle bereits zu Beginn des entsprechenden Teilschrittes. Die Wasserstoffquelle wird vorzugsweise über eine Apparatur in den Teilschritt eingespeist, die dazu geeignet ist die Wasserstoffquelle möglichst fein zu verteilen. Ist die Wasserstoffquelle ein Gas, so kann die Apparatur beispielsweise eine Mischdüse sein. Zur besonders guten Homogenisierung des Gases in dem Gemisch kann es vorteilhaft sein, wenn der Druck in der Prozessschritt mindestens 2, vorzugsweise 4 MPa beträgt. Liegt die Wasserstoffquelle flüssig vor, so kann eine möglichst feine Verteilung z. B. durch die Verwendung von statischen Mischern erzielt werden.

Als Einsatzstoffe können die reinen nicht cyclischen Olefine mit konjugierten Doppelbindungen, Gemische verschiedener solcher Olefine oder Gemische eines oder mehrerer der genannten Olefine mit anderen Kohlenwasserstoffen eingesetzt werden. Bevorzugt wird als Einsatzstoff ein Gemisch von Kohlenwasserstoffen eingesetzt, das ein oder mehrere, vorzugsweise ein nicht cyclisches Olefin mit mindestens zwei konjugierten Doppelbindungen in Mischung mit anderen Kohlenwasserstoffen aufweist.

Besonders bevorzugte Einsatzstoffe weisen als nicht cyclische Olefine mit konjugierten Doppelbindungen 1,3-Butadien und/oder Isopren jeweils entweder als Reinstoff, Gemisch der Reinstoffe oder im Gemisch eines oder beider Olefine mit anderen Kohlenwasserstoffen auf. Ganz besonders bevorzugt werden als Einsatzstoffe Gemische eingesetzt, die zu über 90 Massen-% C₄-Kohlenwasserstoffe und vorzugsweise 1,3-Butadien enthalten.

Als Einsatzstoffe für das erfindungsgemäße Verfahren sind besonders bevorzugt 1,3-Butadienreiche Kohlenwasserstoffströme geeignet. Als Kohlenwasserstoffstrom kann insbesondere ein C₄-Kohlenwasserstoffschnitt eingesetzt werden. Die Kohlenwasserstoffströme können vorzugsweise z. B. Mischungen von 1,3-Butadien mit anderen C₄- und C₃- oder C₅-Kohlenwasserstoffen sein. Solche Mischungen fallen beispielsweise bei Spalt(Crack)-Prozessen zur Produktion von Ethylen und Propylen an, in denen Raffineriegase, Naphtha, Gasöl, LPG (liquified petroleum gas), NGL (natural gas liquid) usw. umgesetzt werden. Die bei den Prozessen als Nebenprodukt anfallenden C₄-Schnitte können neben 1,3-Butadien, Monoolefine (1-Buten, cis-But-2-en, trans-But-2-en, Isobuten), gesättigte Kohlenwasserstoffe (n-Butan, Isobutan), acetylenisch ungesättigte Verbindungen (Ethylacetylen (Butin), Vinylacetylen (Butenin), Methylacetylen (Propin)) sowie allenisch ungesättigte Verbindungen (hauptsächlich 1,2-Butadien) enthalten. Weiterhin können diese Schnitte geringe Mengen an C₃- und C₅-Kohlenwasserstoffen enthalten. Die Zusammensetzung der C₄-Schnitte ist abhängig vom jeweiligen Spaltverfahren, den Betriebsparametern und dem Einsatzstoff. Die Konzentrationen der einzelnen Komponenten liegen bei Steamcrackern typischerweise in folgenden Bereichen:

| Komponente | Massen-% |
|---|---|
| 1,3- Butadien | 25-70 |
| 1-Buten | 9-25 |
| 2-Butene | 4-20 |
| Isobuten | 10-35 |
| n-Butan | 0,5-8 |
| Isobutan | 0,5-6 |
| Σ acetylenischer Verbindungen | 0,05-4 |
| 1,2-Butadien | 0,05-2 |

Im erfindungsgemäßen Verfahren werden vorzugsweise Kohlenwasserstoffgemische mit einem 1,3-Butadiengehalt von größer 35 Massen-% eingesetzt.

Die Einsatzkohlenwasserstoffe können häufig Spuren von Sauerstoff-, Stickstoff-, Schwefel-, Halogen-, insbesondere Chlor- und Schwermetallverbindungen aufweisen, die im erfindungsgemäßen Verfahren störend sein könnten. Es ist daher zweckmäßig, diese Stoffe zunächst abzutrennen. Störende Verbindungen können z. B. Kohlendioxid oder Carbonylverbindungen, wie z. B. Aceton oder Acetaldehyd sein.

Die Abtrennung dieser Verunreinigungen kann z. B. durch Wäschen, insbesondere mit Wasser oder wässrigen Lösungen, oder über Adsorber erfolgen.

Durch eine Wasserwäsche können hydrophile Komponenten, wie beispielsweise Stickstoffkomponenten, aus dem Kohlenwasserstoffgemisch ganz oder teilweise entfernt werden. Beispiele für Stickstoffkomponenten sind Acetonitril oder N-Methylpyrrolidon (NMP). Auch Sauerstoffverbindungen können zum Teil über eine Wasserwäsche entfernt werden. Die Wasserwäsche kann direkt mit Wasser durchgeführt werden oder aber mit wässrigen Lösungen, die z. B. Salze wie z. B. NaHSO₃ aufweisen können (US 3,682,779, US 3,308,201, US 4,125,568, US 3,336,414 oder US 5,122,236).

Es kann vorteilhaft sein, wenn das Kohlenwasserstoffgemisch nach der Wasserwäsche einen Trocknungsschritt durchläuft. Die Trocknung kann nach den im Stand der Technik bekannten Verfahren durchgeführt werden. Beim Vorliegen von gelöstem Wasser kann die Trocknung z. B. unter Verwendung von Molekularsieb als Trocknungsmittel oder durch azeotrope Destillation durchgeführt werden. Freies Wasser kann durch Phasentrennung, z. B. mit einem Koaleszer abgetrennt werden.

Adsorber können eingesetzt werden, um Verunreinigungen im Spurenbereich zu entfernen. Dies kann insbesondere deshalb vorteilhaft sein, weil im Telomerisationsschritt Edelmetallkatalysatoren zum Einsatz kommen, die bereits auf Spuren von Verunreinigungen mit deutlicher Aktivitätsabnahme reagieren. Oftmals werden Stickstoff- oder Schwefelverbindungen über vorgeschaltete Adsorber entfernt. Beispiele für einsetzbare Adsorptionsmittel sind Aluminiumoxide, Molekularsiebe, Zeolithe, Aktivkohle oder mit Metallen imprägnierte Tonerden (z. B. US 4,571,445 oder WO 02/53685). Adsorptionsmittel werden von diversen Firmen vertrieben, beispielsweise von der Firma Alcoa unter dem Namen Selexsorb^{®}, von UOP oder von Axens, z. B. mit den Produktserien SAS, MS, AA, TG, TGS oder CMG.

Mit dem erfindungsgemäßen Verfahren ist es insbesondere möglich Einsatzstoffgemische einzusetzen, die unter anderem auch allenisch ungesättigte Verbindungen und/oder mit einem Anteil von größer-gleich 50 Massen-ppm (wppm), bevorzugt größer-gleich 100 Massen-ppm acetylenisch ungesättigte Verbindungen enthalten. In dem erfindungsgemäßen Verfahren können vorzugsweise nicht cyclische, konjugierte Doppelbindungen aufweisende Olefine enthaltende Gemische als Einsatzstoffe eingesetzt werden, die bis zu 5 Massen-%, vorzugsweise bis zu 3 Massen-% und besonders bevorzugt bis zu 1 Massen-% Alkine bzw. acetylenisch ungesättigte Verbindungen aufweisen. Durch die Verwendbarkeit von Einsatzstoffgemischen, die noch geringe Restmengen an acetylenisch ungesättigten Verbindungen aufweisen, ist es möglich, zahlreiche der technisch anfallenden Kohlenwasserstoffgemische, insbesondere C₄- oder C₅-Kohlenstwasserstoffgemische direkt in dem erfindungsgemäße Verfahren zur Telomerisation als Einsatzstoffe zu verwenden. Sollte ein deutlich höherer Anteil an acetylenisch ungesättigten Verbindungen in einem möglichen Einsatzstoffgemisch vorliegen, so kann es vorteilhaft sein, zumindest einen Teil der vorhandenen acetylenisch ungesättigten Verbindungen (Alkine) vor Einsatz in der Telomerisation, z. B. durch Selektivhydrierung oder Extraktion, aus diesem Gemisch zu entfernen, so dass dann ein Gemisch als Einsatzstoffgemisch eingesetzt werden kann, welches von 0 bis 5 Massen-%, bevorzugt von 50 wppm bis 3 Massen-% und besonders bevorzugt von 100 wppm bis 1 Massen-% an acetylenisch ungesättigten Verbindungen aufweist. Verfahren zur Extraktion oder Selektivhydrierung können dem Stand der Technik entnommen werden.

Die Abtrennung der acetylenischen Verbindungen durch Extraktion ist lange bekannt und ist als Aufarbeitungsschritt ein integraler Bestandteil der meisten Anlagen, die 1,3-Butadien aus Crack-C₄ gewinnen. Ein Verfahren zur extraktiven Abtrennung von acetylenisch ungesättigten Verbindungen aus Crack-C₄, wird beispielsweise in Erdöl und Kohle-Erdgas-Petrochemie vereinigt mit Brennstoffchemie Bd. 34, Heft 8, August 1981, Seite 343 - 346 beschrieben. Bei diesem Verfahren werden in einer ersten Stufe durch Extraktivdestillation mit wasserhaltigem NMP die mehrfach ungesättigten Kohlenwasserstoffe sowie die acetylenisch ungesättigten Verbindungen von den Monoolefinen und gesättigten Kohlenwasserstoffen abgetrennt. Aus dem NMP Extrakt werden die ungesättigten Kohlenwasserstoffe destillativ abgetrennt. Aus dem Kohlenwasserstoffdestillat werden durch eine zweite Extraktivdestillation mit wasserhaltigem NMP die acetylenisch ungesättigten Verbindungen mit 4 C-Atomen abgetrennt. Bei der Aufarbeitung von Crack-C₄ wird durch zwei weitere Destillationen reines 1,3-Butadien abgetrennt, wobei als Nebenprodukte Methylacetylen und 1,2-Butadien anfallen. Im Rahmen des erfindungsgemäßen Verfahrens kann das hier beschriebene mehrstufige Verfahren als Vorbehandlung für das Einsatzstoffgemisch durchgeführt werden, wobei auf die destillative Abtrennung des 1,2-Butadiens verzichtet werden kann.

Optional kann die Abtrennung von acetylenischen Verbindungen aus einem 1,3-Butadien-haltigen Strom unter Verwendung von einer oder mehreren ionischen Flüssigkeit(en), z. B. als Extraktionsmittel, erfolgen.

Die durch Extraktion erhaltenen Kohlenwasserstoffströme, die vorzugsweise weniger als 5-Massen-% an acetylenischen Verbindungen enthalten, können besonders bevorzugt direkt als Einsatzstoff in dem erfindungsgemäßen Verfahren eingesetzt werden.

Die teilweise Entfernung der acetylenisch ungesättigten Verbindungen aus dem einzusetzenden Kohlenwasserstoffstrom durch Selektivhydrierung der acetylenisch ungesättigten Verbindungen in Gegenwart von Dienen und Monoolefine kann z. B. an kupferhaltigen, palladiumhaltigen oder Mischkatalysatoren durchgeführt werden.

Falls acetylenisch ungesättigte Verbindungen vorhanden sind, enthalten die im erfindungsgemäßen Verfahren eingesetzten Einsatzstoffe, insbesondere bei der Verwendung von 1,3-Butadien aufweisenden C₄-Kohlenwasserstoffgemischen, als acetylenisch ungesättigte Verbindungen (Alkine), die bevorzugt ausgewählt sind aus Vinylacetylen und/oder 1-Butin.

Beim Vorhandensein von Alkinen im Einsatzstoffgemisch wird durch die Wasserstoffquelle vorzugsweise soviel Wasserstoff dem Gesamtprozess zugeführt, dass das molare Verhältnis von Wasserstoff zu Alkin zumindest 1 zu 1, bevorzugt von 1 zu 1 bis 2 zu 1, besonders bevorzugt von 1 zu 1 bis 1,5 zu 1 und ganz besonders bevorzugt von 1 zu 1 bis 1,1 zu 1 beträgt. Eine deutliche Überschreitung dieser Werte führt zu einem zunehmenden Verlust der nicht cyclischen, konjugierte Doppelbindungen aufweisenden Olefine, wie z. B. des 1,3-Butadiens. Die Konzentration des Alkins in dem Einsatzstoffgemisch kann kontinuierlich oder in regelmäßigen Abständen, z. B. gaschromatographisch bestimmt werden.

In dem erfindungsgemäßen Verfahren können alle für die Telomerisation geeigneten Katalysatoren eingesetzt werden. Als Katalysator für die Telomerisation werden bevorzugt Metallkomplexe der Metalle Palladium (Pd), Eisen (Fe), Ruthenium (Ru), Osmium (Os), Kobalt (Co), Rhodium (Rh), Iridium (Ir), Nickel (Ni) oder Platin (Pt) eingesetzt. Als Liganden können z. B. Phosphorliganden, wie z. B. Phosphine, Phosphinine, Phosphinite, Phosphonite oder Phosphite, wie z. B. Triphenylphosphine oder Carbenliganden eingesetzt werden, wobei es auch vorteilhaft sein kann unterschiedliche Liganden gleichzeitig zu verwenden. Besonders bevorzugt werden Metallcarbenkomplexe als Katalysatoren eingesetzt.

Ganz besonders bevorzugt werden Palladiumverbindungen, insbesondere Palladiumcarbenkomplexe als Katalysatoren im Telomerisationsschritt verwendet. Die Liganden in den als Katalysator eingesetzten Metallkomplexen sind besonders bevorzugt trivalente Phosphorverbindungen oder Carbene.

Besonders bevorzugt werden Metallkomplexe als Katalysator verwendet, die mindestens ein durch Heteroatome stabilisiertes Carben als Ligand aufweisen. Beispiele für derartige Liganden werden unter anderem in den Dokumenten DE 101 28 144, DE 101 49 348, DE 101 48 722, DE 100 62 577, EP 1 308 157 und WO 01/66248 beschrieben. Diese Dokumente und insbesondere die dort beschriebenen Liganden zählen zum Offenbarungsgehalt der vorliegenden Anmeldung. Darüber hinaus kann der aktive Komplex noch weitere Liganden aufweisen. Die Carbenliganden können offene Liganden oder cyclische Liganden sein.

Vorzugsweise wird im erfindungsgemäßen Verfahren ein Palladiumcarbenkomplex als Telomerisationskatalysator eingesetzt, der einen Carbenliganden der allgemeinen Formel (VIII) 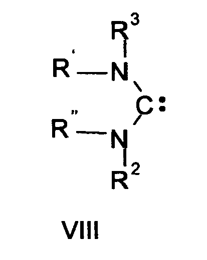 mit R², R", R' und R³ gleich oder unterschiedlich Wasserstoff oder Kohlenwasserstoffgruppen, wobei die Kohlenwasserstoffgruppen gleiche oder unterschiedliche, lineare, verzweigte oder zyklische Reste ausgewählt aus der Gruppe von Alkylresten mit 1 bis 50 Kohlenstoffatomen, Alkenylresten mit 2 bis 50 Kohlenstoffatomen, Alkinylresten mit 2 bis 50 Kohlenstoffatomen und Arylresten mit 6 bis 30 Kohlenstoffatomen, in welchen zumindest ein Wasserstoffatom durch eine funktionelle Gruppe ersetzt sein kann, sein können und/oder R² und R" und/oder R' und R³ gleich Teil eines cyclischen Systems welches gleich oder unterschiedlich ist, welches ein Kohlenstoffgerüst mit von 2 bis 20 Kohlenstoffatomen und ein Stickstoffatom gemäß Formel VIII aufweist, wobei die Kohlenstoffatome von R² und R" und/oder R' und R³ nicht gezählt werden und wobei zumindest ein Wasserstoffatom in dem cyclischen System durch eine funktionelle Gruppe ersetzt sein kann und/oder zumindest ein Kohlenstoffatom des cyclischen Systems durch ein Heteroatom, ausgewählt aus der Gruppe bestehend aus S, P, O und N, ersetzt sein kann,
und/oder R² und/oder R" und/oder R' und/oder R³ sind durch eine Brücke aus 1 bis 20 Kohlenstoffatomen mit einem Liganden L verbunden, wobei die Kohlenstoffatome der Reste R², R", R' und R³ nicht mitgezählt werden,
und L ein weiterer Ligand ist, der für einen neutralen Zweielektronen-Donor, Teil eines cyclischen Systems und/oder einen anionischen Liganden steht, wobei die funktionellen Gruppen z. B. ausgewählt sein können aus den Gruppen: -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H und -PO₃H₂, wobei die Alkylgruppen z. B. 1 bis 24 und die Arylgruppen z. B. 5 bis 24 Kohlenstoffatome beinhalten können, aufweist. Die Herstellung solcher Liganden kann z. B. DE 101 48 722 entnommen werden.

Bevorzugt wird im erfindungsgemäßen Verfahren ein Palladiumcarbenkomplex als Telomerisationskatalysator eingesetzt, der einen Carbenliganden der allgemeinen Formel (VIII) aufweist mit
- R²; R³:: gleich oder verschieden lineare, verzweigte, substituierte oder unsubstituierte cyclische oder alicyclische Alkylgruppen mit 1 bis 24 Kohlenstoffatomen, oder substituierte oder unsubstituierte, mono- oder polycyclische Arylgruppen mit 6 bis 24 Kohlenstoffatomen oder mono- oder polycyclischer, substituierter oder unsubstituierter Heterocyclus mit 4 bis 24 Kohlenstoffatomen und mindestens einem Heteroatom aus der Gruppe N, O, S,
- R', R":: gleich oder verschiedenen Wasserstoff, Alkyl, Aryl, Heteroaryl, -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, -PO₃H₂, wobei die Alkylgruppen 1-24 und die Aryl- und Heteroarylgruppen 5 bis 24 Kohlenstoffatome beinhalten und die Reste R' und R" auch Teil eines verbrückenden aliphatischen oder aromatischen Rings sein können.

Ganz besonders bevorzugt werden Carbenliganden eingesetzt, die einen 5-Ring aufweisen. Vorzugsweise im erfindungsgemäßen Verfahren eingesetzte, einen 5-Ring aufweisende Liganden sind z. B. solche der Formeln IX, X, XI und XII 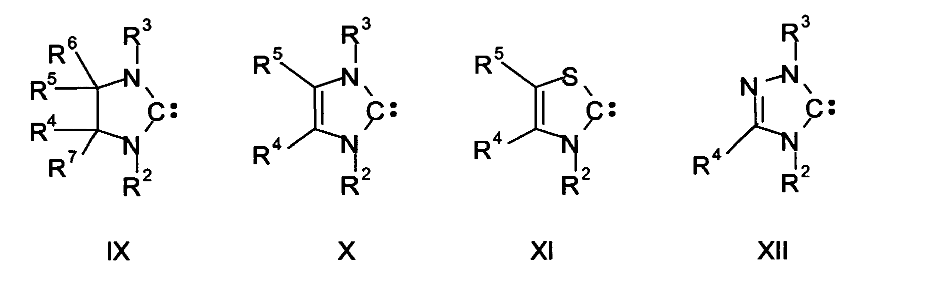 mit
- R²; R³:: gleich oder verschieden lineare, verzweigte, substituierte oder unsubstituierte cyclische oder alicyclische Alkylgruppen mit 1 bis 24 Kohlenstoffatomen, oder substituierte oder unsubstituierte, mono- oder polycyclische Arylgruppen mit 6 bis 24 Kohlenstoffatomen oder mono- oder polycyclischer, substituierter oder unsubstituierter Heterocyclus mit 4 bis 24 Kohlenstoffatomen und mindestens einem Heteroatom aus der Gruppe N, O, S,
- R⁴, R⁵, R⁶, R⁷:: gleich oder verschiedenen Wasserstoff, Alkyl, Aryl, Heteroaryl, -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, -PO₃H₂, wobei die Alkylgruppen 1-24 und die Aryl- und Heteroarylgruppen 5 bis 24 Kohlenstoffatome beinhalten und die Reste R⁴, R⁵, R⁶ und R⁷ auch Teil eines verbrückenden aliphatischen oder aromatischen Rings sein können.

Beispiele für Carbenliganden, die den allgemeinen Formeln IX oder X entsprechen, und Komplexe, die derartige Liganden enthalten, sind in der Fachliteratur bereits beschrieben (W. A. Herrmann, C. Köcher, Angew. Chem. 1997, 109, 2257*;* Angew. Chem. Int. Ed. Engl. 1997, 36, 2162; V.P.W. Böhm, C.W.K. Gstöttmayr, T. Weskamp, W.A. Herrmann, J. Organomet. Chem. 2000, 595, 186; DE 44 47 066).

Die Reste R² und R³ können insbesondere für einen mono- oder polycyclischen Ring stehen, der mindestens ein Heteroatom ausgewählt aus den Elementen Stickstoff, Sauerstoff und Schwefel enthält und gegebenenfalls weitere Substituenten ausgewählt aus den Gruppen -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -Aryl-, -Alkyl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, - PO₃H₂ aufweist Die Alkylgruppen weisen 1 bis 24 und die Arylgruppen 5 bis 24 Kohlenstoffatome auf. In dem Fall, das Pd als Metall der 8. bis 10. Gruppe des Periodensystems verwendet wird, weist einer oder beide Liganden R² und R³, bevorzugt diese Bedeutungen auf.

Die Reste R², R³, R⁴, R⁵, R⁶ und/oder R⁷ können jeweils gleich oder verschieden sein und mindestens einen Substituenten aus der Gruppe -H, -CN, -COOH, -COO-Alkyl, -COO-Aryl, -OCO-Alkyl, -OCO-Aryl, -OCOO-Alkyl, -OCOO-Aryl, -CHO, -CO-Alkyl, -CO-Aryl, -Aryl, -Alkyl, -Alkenyl, -Allyl, -O-Alkyl, -O-Aryl, -NH₂, -NH(Alkyl), -N(Alkyl)₂, -NH(Aryl), -N(Alkyl)₂, -F, -Cl, -Br, -I, -OH, -CF₃, -NO₂, -Ferrocenyl, -SO₃H, -PO₃H₂ aufweisen, wobei die Alkylgruppen 1 bis 24, bevorzugt 1 bis 20, die Alkenylgruppen 2 bis 24, die Allylgruppen 3 bis 24 und die mono- oder polycyclischen Arylgruppen 5 bis 24 Kohlenstoffatome beinhalten.

Die Reste R₄ bis R₆ können z. B. über (CH₂)- oder (CH)-Gruppen miteinander kovalent verknüpft sein.

Bei Substituenten mit aciden Wasserstoffatomen können die Protonen durch Metall- oder Ammoniumionen ersetzt sein.

Die Reste R² und R³ können besonders bevorzugt Reste sein, die sich von fünf- und sechsgliedrigen Heteroalkanen, Heteroalkenen und Heteroaromaten wie 1,4-Dioxan, Morpholin, γ-Pyran, Pyridin, Pyrimidin, Pyrazin, Pyrrol, Furan, Thiophen, Pyrazol, Imidazol, Thiazol und Oxazol ableiten. In der nachfolgenden Tabelle 1 sind konkrete Beispiele für derartige Reste R² und R³ widergegeben. Darin bezeichnet ~ jeweils den Anknüpfungspunkt zu dem Fünfring-Heterozyklus bzw. der Verbindung gemäß Formel VIII.

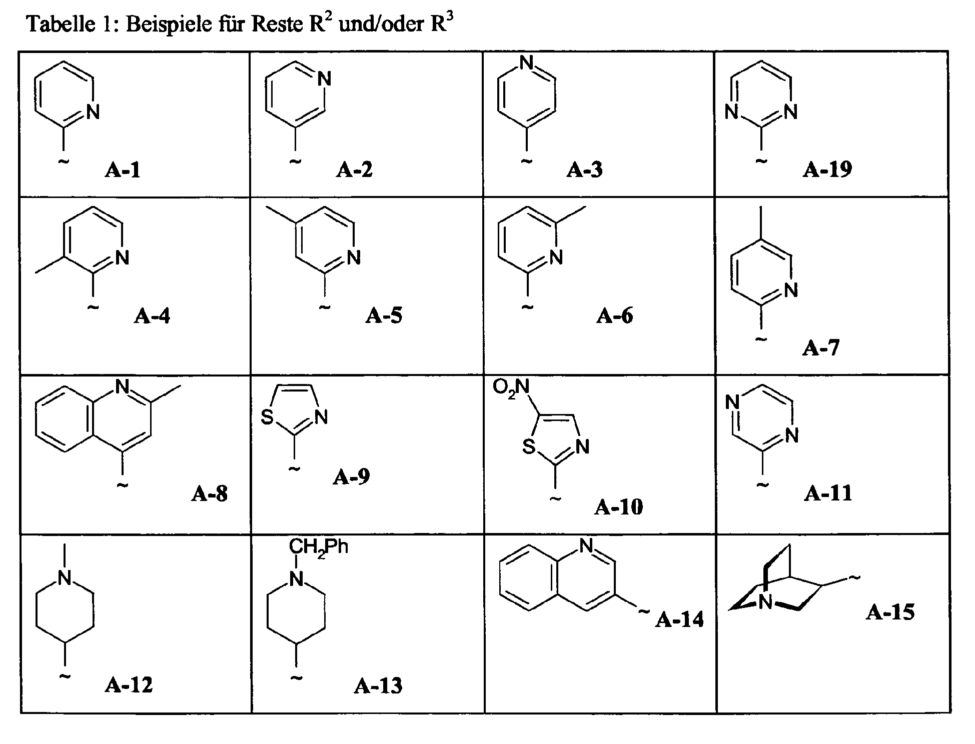

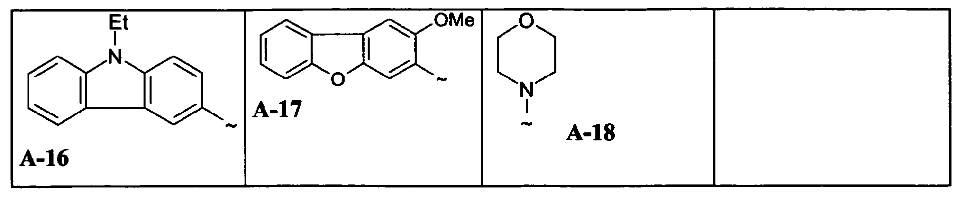

Im Rahmen dieser Erfindung werden unter Carbenliganden sowohl freie Carbene, die als Ligand fungieren können, als auch an Metall koordinierte Carbene verstanden.

Das Katalysatormetall, insbesondere das als Katalysatormetall verwendete Palladium, aus dem sich unter Reaktionsbedingungen der aktive Katalysator bildet, kann auf verschiedene Weisen in den Prozess eingebracht werden.

Das Metall (Palladium) kann
a) als Metall-Carbenkomplex (Palladium-Carbenkomplex), wobei das Metall (Palladium) bevorzugt in den Oxidationsstufen (II) oder (0) vorliegt oder
b) in Form von Metallvorstufen (Palladiumvorstufen), aus denen in situ die Katalysatoren gebildet werden,
in den Prozess eingebracht werden.

### Zu a)

Beispiele sind Palladium(0)carben-olefin-Komplexe, Palladium(0)dicarbenkomplexe und Palladium(II)dicarbenkomplexe, Palladium(0)carben-1,6-dien-Komplexe. Als 1,6-Dien können beispielsweise Diallylamin, 1,1'-Divinyltetramethyldisiloxan, 2,7-Octadienylether oder 2,7-Octadienylamine fungieren. Weitere Beispiele zeigen die nachfolgenden Formeln I-a bis I-e.

| | | |
|---|---|---|
| 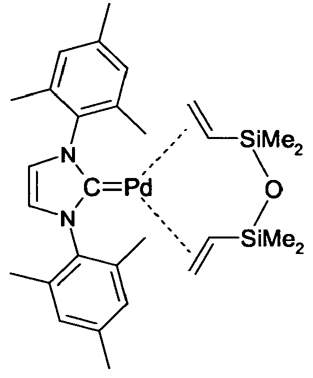 | 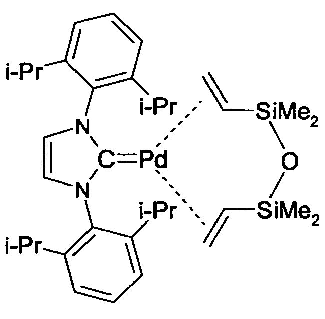 | 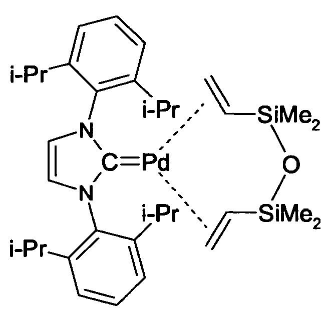 |
| I-a | I-b | I-c |
| 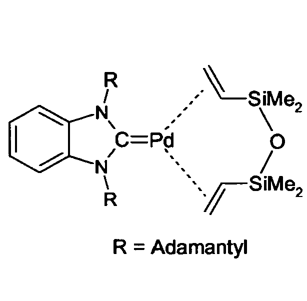 | 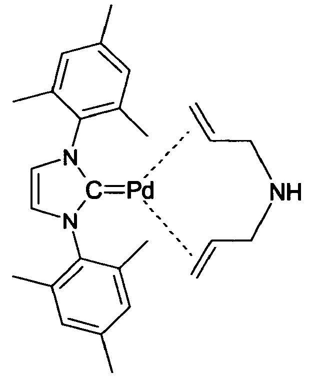 | 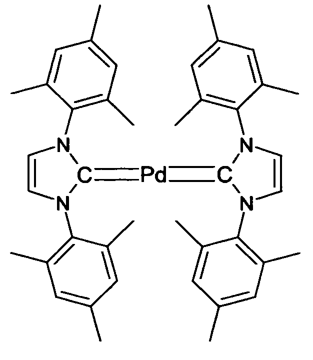 |
| I-d | I-e | I-f |
| 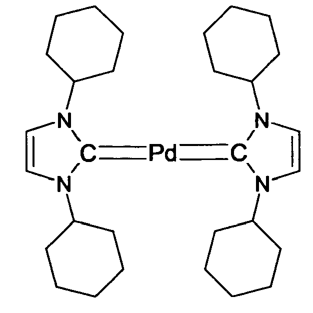 | 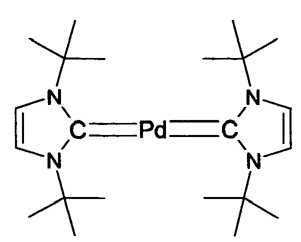 | 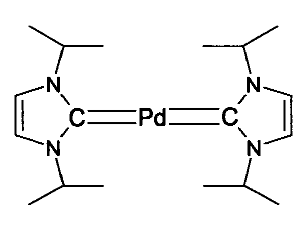 |
| I-g | I-h | I-i |
| 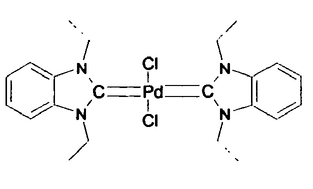 | 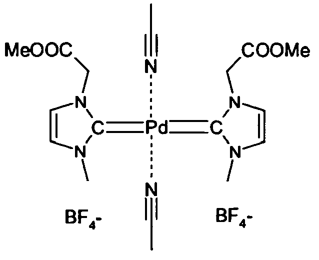 | 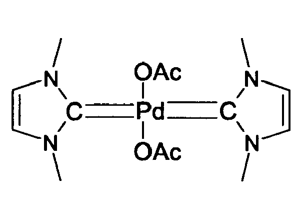 |
| I-j | I-k | I-l |

Die Carbenkomplexe des Palladiums können auf verschiedenste Weisen dargestellt werden. Ein einfacher Weg ist beispielsweise die Anlagerung von Carbenliganden oder der Austausch von Liganden an Palladiumkomplexen durch Carbenliganden. So sind beispielsweise die Komplexe I-f bis I-i durch Austausch der Phosphorliganden des Komplexes Bis(tri-o-tolylphosphin)palladium(0) erhältlich (T. Weskamp, W.A. Herrmann, J. Organomet. Chem. 2000, 595, 186).

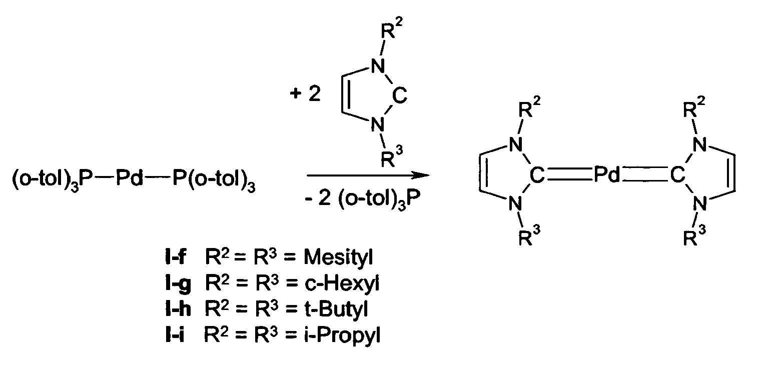

### Zu b)

Als Palladiumvorstufen können beispielsweise eingesetzt werden: Palladium(II)acetat, Palladium(II)chlorid, Palladium(II)bromid, Lithiumtetrachloropalladat, Palladium(II)acetylacetonat, Palladium(0)-dibenzylidenaceton-Komplexe, Palladium(II)propionat, Palladium(II)-chloridbisacetonitril, Palladium(II)-bistriphenylphosphandichlorid, Palladium(II)chloridbis-benzonitril, Bis(tri-o-tolylphosphin)palladium(0) und weitere Palladium(0)- und Palladium(II)-Komplexe.

Die Carbene nach den allgemeinen Formeln IX und X können in Form freier Carbene oder als Metallkomplexe eingesetzt werden oder in situ aus Carbenvorstufen erzeugt werden.

Als Carbenvorstufen eignen sich beispielsweise Salze der Carbene gemäß den allgemeinen Formeln XIII und XIV, 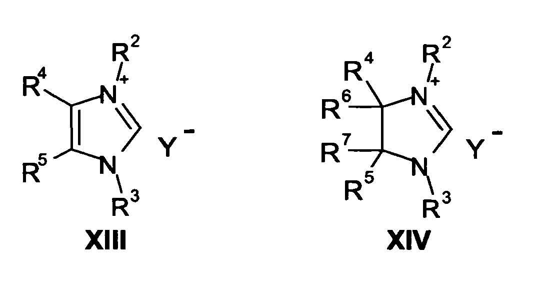 wobei R², R³, R⁴, R⁵, R⁶, R⁷ dieselbe Bedeutung haben wie in Formeln IX und X und Y für eine einfach geladene anionische Gruppe oder entsprechend der Stöchiometrie anteilig für eine mehrfach geladene anionische Gruppe steht

Beispiele für Y sind Halogenide, Hydrogensulfat, Sulfat, Alkylsulfate, Arylsulfate, Borate, Hydrogencarbonat, Carbonat, Alkylcarboxylate, Arylcarboxylate.

Aus den Salzen der Carbene können die entsprechenden Carbene beispielsweise durch Umsetzung mit einer Base freigesetzt werden.

Die Konzentration des Katalysators, formal angegeben in ppm (Masse) an Katalysator-Metall, insbesondere Palladium-Metall bezogen auf die Gesamtmasse, beträgt im erfindungsgemäßen Verfahren vorzugsweise 0,01 ppm bis 1000 ppm, bevorzugt 0,5 bis 100 ppm, besonders bevorzugt 1 bis 50 ppm. Das Verhältnis [Mol/Mol] von Ligand, vorzugsweise Carben zu Metall, insbesondere das Verhältnis von Carben zu Pd beträgt im Reaktionsgemisch vorzugsweise 0,01 : 1 bis 250 : 1, bevorzugt 1 : 1 bis 100 : 1, besonders bevorzugt 1 : 1 bis 50:1. Neben den Carbenliganden können noch weitere Liganden, beispielsweise Phosphorliganden, wie z. B. Triphenylphosphin, in der Reaktionsmischung vorliegen.

Als Nukleophil (VII) werden bevorzugt Verbindungen der allgemeinen Formeln

R^{1a}-O-H (VIIa)

oder

(R^{1a})(R^{1b})N-H (VIIb)

oder

R^{1a}-COOH (VIIc)

worin R^{1a} und R^{1b} unabhängig voneinander ausgewählt sind aus Wasserstoff, einer linearen, verzweigten oder cyclischen C₁ bis C₂₂-Alkylgruppe, einer Alkenylgruppe, einer Alkinylgruppe, einer C₅ bis C₁₈ Arylgruppe oder einer -CO-Alkyl-(C₁-C₈)-Gruppe oder einer -CO-Aryl-(C₅-C₁₀)-Gruppe, wobei diese Gruppen Substituenten ausgewählt aus der Gruppe -CN, -COOH, - COO-Alkyl-(C₁-C₈), -CO-Alkyl-(C₁-C₈), -Aryl-(C₅-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alky12-(C₁-C₈), -CHO, -SO₃H, -NH₂, -F, - Cl, -OH, -CF₃, -NO₂ enthalten können, und wobei die Reste R^{1a} und R^{1b} über kovalente Bindungen miteinander verknüpft sein können, eingesetzt. Vorzugsweise werden als Nucleophile solche Verbindungen eingesetzt, bei denen die Reste R^{1a} und gegebenenfalls R^{1b} gleich Wasserstoff, Methyl, Ethyl, n-Propyl, iso-Propyl, tert.-Butyl, n-Butyl, sec.-Butyl, Pentyl, Hexyl, Heptyl, Octyl, Octenyl, Octadienyl, Isononyl, 2-Ethylhexyl, n-Nonyl, Phenyl, m-, o-oder p-Methylphenyl, Naphtyl, 2,4-Di-tert.-butylphenyl, 2,6-Di-tert.-butylmethylphenyl, Hydrogencarbonyl, Methylcarbonyl, Ethylcarbonyl, Propylcarbonyl oder Phenylcarbonyl sind.

Besonders bevorzugt werden als Nucleophile (VII) Wasser, Alkohole, Phenole, Polyole, Carbonsäuren, Ammoniak und/oder primäre oder sekundäre Amine eingesetzt. Speziell sind dies:
- Wasser, Ammoniak,
- Monoalkohole und Phenole wie zum Beispiel Methanol, Ethanol, n-Propanol, Isopropanol, Allylalkohol, n-Butanol, i-Butanol, Octanol, 2-Ethylhexanol, Isononanol, Benzylalkohol, Cyclohexanol, Cyclopentanol oder 2,7-Octadien-1-ol, Phenol,
- Dialkohole wie zum Beispiel Ethylenglykol, 1,2-Propandiol, 1,3-Propandiol, 1,4-Butandiol, 1,2-Butandiol, 2,3-Butandiol und 1,3-Butandiol,
- Hydroxyverbindungen wie zum Beispiel α-Hydroxyessigsäureester,
- primäre Amine wie zum Beispiel Methylamin, Ethylamin, Propylamin, Butylamin, Octylamin, 2,7-Octadienylamin, Dodecylamin, Ethylendiamin oder Hexamethylendiamin,
- sekundäre Amine wie Dimethylamin, Diethylamin, N-Methylanilin, Bis(2,7-octadienyl)amin, Dicyclohexylamin, Methylcyclohexylamin, Pyrrolidin, Piperidin, Morpholin, Piperazin oder Hexamethylenimin oder
- Carbonsäuren wie Ameisensäure, Essigsäure, Propansäure, Butensäure, Isobutensäure, Benzoesäure, 1,2 Benzoldicarbonsäure (Phthalsäure).

Ganz besonders bevorzugt werden als Nucleophile (VII) im Telomerisationsschritt Methanol, Ethanol, 2-Ethylhexanol, Octanol, Octenol, Octadienol, Isopropanol, n-Propanol, Isobutanol, n-Butanol, Isononanol, Ameisensäure, Essigsäure, Propionsäure, n-Butansäure, iso-Butansäure, Benzoesäure, Phthalsäure, Phenol, Dimethylamin, Methylamin, Ammoniak und/oder Wasser eingesetzt. Vorteilhafterweise wird als Nucleophil Methanol eingesetzt.

Nucleophile, die selbst über eine Telomerisationsreaktion erhalten werden können, können direkt eingesetzt oder aber in situ gebildet werden. So kann beispielsweise 2,7-Octadien-1-ol aus Wasser und Butadien in Anwesenheit des Telomerisationskatalysators in situ gebildet werden, 2,7-Octadienylamin aus Ammoniak und 1,3-Butadien usw.

Für das Verhältnis von Nucleophil zum Ausgangsolefin mit mindestens zwei konjugierten Doppelbindungen in der Telomerisationsreaktion ist die Anzahl der aktiven Wasserstoffatome im Telogen zu berücksichtigen. So hat beispielsweise Methanol ein aktives Wasserstoffatom, Ethylenglykol hat zwei, Methylamin hat zwei usw.

Pro Mol aktiven Wasserstoffatom des Nucleophils, das mit dem Ausgangsolefin reagieren kann, werden bevorzugt 0,001 Mol bis 10 Mol Ausgangsolefin in der Telomerisationsreaktion eingesetzt. Bei einer Reaktionsführung mit einer flüssigen Phase wird ein Verhältnis von 0,1 Mol bis 2 Mol Ausgangsolefin pro Mol aktivem Wasserstoff besonders bevorzugt.

Es kann vorteilhaft sein, wenn das erfindungsgemäße Verfahren in Anwesenheit eines Lösemittels durchgeführt wird. Als Lösemittel für die Telomerisationsreaktion kann das eingesetzte Nucleophil, wenn es bei Reaktionsbedingungen als Flüssigkeit vorliegt, und/oder inerte, organische Lösemittel eingesetzt werden. Bevorzugt wird der Zusatz von Lösemitteln bei Einsatz von Nucleophilen, die unter Reaktionsbedingungen als Feststoffe vorliegen oder bei Produkten, die unter den Reaktionsbedingungen als Feststoffe anfallen würden. Geeignete Lösemittel sind unter anderem aliphatische, cycloaliphatische und aromatische Kohlenwasserstoffe wie zum Beispiel C₃-C₂₀-Alkane, Mischungen niederer Alkane (C₃-C₂₀), Cyclohexan, Cyclooctan, Ethylcyclohexan, Alkene und Polyene, Vinylcyclohexen, 1,3,7-Octatrien, die C₄-Kohlenwasserstoffe aus Crack-C₄-Schnitten, Benzol, Toluol und Xylol; polare Lösemittel wie zum Beispiel tertiäre und sekundäre Alkohole, Amide wie zum Beispiel Acetamid, Dimethylacetamid und Dimethylformamid, Nitrile wie zum Beispiel Acetonitril und Benzonitril, Ketone wie zum Beispiel Aceton, Methylisobutylketon und Diethylketon; Carbonsäureester wie zum Beispiel Essigsäureethylester, Ether wie beispielsweise Dipropylether, Diethylether, Dimethylether, Methyloctylether, Methyltertiärbutylether, Ethyltertiärbutylether 3-Methoxyoctan, Dioxan, Tetrahydrofuran, Anisol, Alkyl- und Arylether von Ethylenglycol, Diethylenglycol, Triethylenglycol, Tetraethylenglycol, Polyethylenglycol, Propylenglycol, Dipropylenglycol, Tripropylenglycol und Polypropylenglycol und andere polare Lösemittel wie zum Beispiel Sulfolan, Dimethylsulfoxid, Ethylencarbonat, Propylencarbonat und Wasser. Auch Ionische Flüssigkeiten, beispielsweise Imidazolium- oder Pyridiniumsalze, können als Lösemittel eingesetzt werden. Die Lösemittel können allein oder als Mischungen verschiedener Lösemittel eingesetzt werden.

Die Temperatur, bei der die Telomerisationsreaktion ausgeführt wird, liegt vorzugsweise im Bereich von 10 bis 180 °C, bevorzugt im Bereich von 30 bis 120 °C und besonders bevorzugt im Bereich von 40 bis 100 °C. Der Reaktionsdruck beträgt vorzugsweise von 1 bis 300 bar, bevorzugt von 1 bis 120 bar, besonders bevorzugt von 1 bis 64 bar und ganz besonders bevorzugt von 1 bis 20 bar.

Oftmals ist es vorteilhaft, die Telomerisationsreaktion in Gegenwart von Basen durchzuführen. Bevorzugt werden basische Komponenten mit einem pK_{b}-Wert kleiner 7, insbesondere Verbindungen ausgewählt aus der Gruppe der Amine, Alkoholate, Phenolate, Alkalimetallsalze oder Erdalkalimetallsalze eingesetzt.

Als basische Komponente sind beispielsweise geeignet Amine wie Trialkylamine, die alicyclisch oder/und offenkettig sein können, Amide, Alkali- oder/und Erdalkalisalze aliphatischer oder/und aromatischer Carbonsäuren, wie Acetate, Propionate, Benzoate bzw. entsprechende Carbonate, Hydrogencarbonate, Alkoholate von Alkali- und/oder Erdalkalielementen, Phosphate, Hydrogenphosphate oder/und Hydroxide bevorzugt von Lithium, Natrium, Kalium, Calcium, Magnesium, Cäsium, Ammonium- und Phosphoniumverbindungen. Bevorzugt sind als Zusatz Hydroxide der Alkali- und Erdalkalielemente und Metallsalze des Nucleophils nach den allgemeinen Formeln III, IV oder V.

Vorzugsweise werden von der basischen Komponente von 0,01 Mol-% bis 10 Mol-% (bezogen auf das Ausgangsolefin), bevorzugt von 0,1 Mol-% bis 5 Mol-% und ganz besonders bevorzugt von 0,2 Mol-% bis 1 Mol-% eingesetzt.

Die Telomerisation kann kontinuierlich oder diskontinuierlich betrieben werden und ist nicht auf den Einsatz bestimmter Reaktortypen begrenzt. Beispiele für Reaktoren, in denen die Reaktion durchgeführt werden kann, sind Rührkesselreaktor, Rührkesselkaskade, Strömungsrohr und Schlaufenreaktor. Auch Kombinationen verschiedener Reaktoren sind möglich, beispielsweise ein Rührkesselreaktor mit nachgeschaltetem Strömungsrohr.

Die Telomerisation kann, um eine hohe Raum-Zeit-Ausbeute zu erhalten, vorzugsweise nicht bis zum vollständigen Umsatz des Einsatzolefins durchgeführt werden. Dies gilt insbesondere dann, wenn das Einsatzolefin 1,3-Butadien ist. In diesem Fall ist es bevorzugt den Umsatz auf maximal 95 %, besonders bevorzugt auf 88 % zu begrenzen.

Die erfindungsgemäße Zugabe von Wasserstoff über eine Wasserstoffquelle zu einem in einem Teilschritt vorliegenden Prozessgemisch kann in einem oder mehreren Teilschritten erfolgen. Weist das erfindungsgemäße Telomerisationsverfahren nur einen Teilschritt, nämlich die Telomerisationsreaktion selbst auf, so erfolgt die Zugabe des Wasserstoffs natürlich durch Zugabe der Wasserstoffquelle zu diesem (Teil-)Schritt. Vorzugsweise sind in dem erfindungsgemäßen Verfahren noch ein oder mehrere weitere Teilschritt vorhanden. Solche Teilschritte können z. B. ein Schritt der Abtrennung des Katalysators und gegebenenfalls ein Teilschritt der Rückführung des Katalysators in den Reaktor sein. Die Wasserstoffquelle wird vorzugsweise dem Gesamtprozess der Telomerisation im Reaktionsschritt zugegeben wird. Die Zugabe von Wasserstoff über die Wasserstoffquelle im Reaktionsschritt hat den Vorteil, dass die Hemmung der Telomerisation durch acetylenisch ungesättigte Verbindungen direkt im Reaktionsgemisch verhindert werden kann. Ein Nachteil der Zugabe des Wasserstoffs im Reaktionsschritt kann aber sein, dass Edukte oder Produkte zu unerwünschten Nebenprodukten hydriert werden können und es so zu Ausbeuteverlusten kommen kann. Dies ist insbesondere dann der Fall, wenn die Telomerisation mit einem Überschuss an Wasserstoff im Vergleich zu den vorhandenen acetylenisch ungesättigten Verbindungen durchgeführt wird.

Weist der Gesamtprozess der Telomerisation einen Schritt der Katalysatorrückführung auf, so wird die Wasserstoffquelle dem Gesamtprozess der Telomerisation bevorzugt im Teilschritt der Katalysatorrückführung zugegeben. Die Zugabe der Wasserstoffquelle in die Katalysatorrückführung hat den Vorteil, dass das Produkt und auch das Edukt weitestgehend vom Katalysator in einem Vorschritt abgetrennt wurde und somit der über die Wasserstoffquelle zugegebene Wasserstoff nicht wertvolles Edukt oder Produkt zu unerwünschten Nebenprodukten hydriert und somit die Ausbeute nicht verringert wird. Es kann vorteilhaft sein, wenn die Wasserstoffquelle sowohl dem Teilschritt der Reaktion als auch dem Teilschritt der Katalysatorrückführung zugegeben wird

Das erfindungsgemäße Verfahren kann insbesondere zur Herstellung einer Verbindung gemäß der Formel II 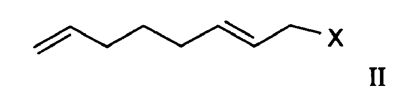 in der X ein Rest OR^{1a} oder NR^{1a}R^{1b} wobei R^{1a} und R^{1b} unabhängig voneinander ausgewählt sind aus Wasserstoff, einer linearen, verzweigten oder cyclischen C₁ bis C₂₂-Alkylgruppe, einer Alkenylgruppe, einer Alkinylgruppe, einer C₅ bis C₁₈ Arylgruppe oder einer -CO-Alkyl-(C₁-C₈)-Gruppe oder einer -CO-Aryl-(C₅-C₁₀)-Gruppe, wobei diese Gruppen Substituenten ausgewählt aus der Gruppe -CN, -COOH, -COO-Alkyl-(C₁-C₈), -CO-Alkyl-(C₁-C₈), -Aryl-(C₅-C₁₀), -COO-Aryl-(C₆-C₁₀), -CO-Aryl-(C₆-C₁₀), -O-Alkyl-(C₁-C₈), -O-CO-Alkyl-(C₁-C₈), - N-Alkyl₂-(C₁-C₈), -CHO, -SO₃H, -NH₂, -F, -Cl, -OH, -CF₃, -NO₂ enthalten können, und wobei die Reste R^{1a} und R^{1b} über kovalente Bindungen miteinander verknüpft sein können, aus einem

### 1,3-Butadien-haltigen Kohlenwasserstoffstrom eingesetzt werden.

Mit dem erfindungsgemäßen Verfahren können insbesondere Verbindungen der IIIa oder IIIb, 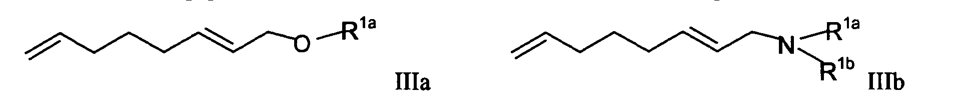 durch Umsetzung von 1,3-Butadien mit einem Nucleophil (VII) gemäß den Formeln VIIa, VIIb oder VIIc

R^{1a}-O-H VIIa

(R^{1a})(R^{1b})N-H VIIb

R^{1a}-COOH VIIc

hergestellt werden, wobei R^{1a} und R^{1b} die oben genannte Bedeutung haben.

Besonders bevorzugt werden mit dem erfindungsgemäßen Verfahren Verbindungen der Formel II hergestellt, in denen das X für OR^{1a} oder NR^{1a}R^{1b} steht, mit
R^{1a} gleich H, Methyl, Ethyl, n-Propyl, iso-Propyl, tert.-Butyl, n-Butyl, sec.-Butyl, Pentyl, Hexyl, Heptyl, Octyl, Octenyl, Octadienyl, Isononyl, 2-Ethylhexyl, n-Nonyl, Phenyl, m-, o-oder p-Methylphenyl, Naphtyl, 2,4-Di-tert.-butylphenyl, 2,6-Di-tert.-butylmethylphenyl, Hydrogencarbonyl, Methylcarbonyl, Ethylcarbonyl, Propylcarbonyl oder Phenylcarbonyl und/oder
R^{1b} gleich H, Methyl, Ethyl, n-Propyl, iso-Propyl, tert.-Butyl, n-Butyl, sec.-Butyl, Pentyl, Hexyl, Heptyl, Octyl, Octenyl, Octadienyl, Isononyl, 2-Ethylhexyl, n-Nonyl, Phenyl, m-, o-oder p-Methylphenyl, Naphtyl, 2,4-Di-tert.-butylphenyl, 2,6-Di-tert.-butylmethylphenyl, Hydrogencarbonyl, Methylcarbonyl, Ethylcarbonyl, Propylcarbonyl oder Phenylcarbonyl. Ganz besonders bevorzugt wird mit dem erfindungsgemäßen Verfahren eine Verbindung gemäß der Formel IIIa mit R^{1a} = Wasserstoff, Methyl, Ethyl, Phenyl oder Methylcarbonyl hergestellt. Die Verbindungen der Formeln IIIa und IIIb können sowohl in der cis- als auch in der trans-Form vorliegen.

Der Austrag aus dem Reaktionsschritt der Telomerisation kann z. B. hauptsächlich das Telomerisationsprodukt, Nebenprodukte, "inerte Kohlenwasserstoffe", Restmengen an Einsatzolefin, Restmengen an Nucleophil und Katalysatorsystem (Katalysatormetall, Liganden und gegebenenfalls Basen etc.) bzw. dessen Folgeprodukte und gegebenenfalls zugesetzte Lösemittel aufweisen bzw. daraus bestehen. Die Auftrennung des Austrags aus dem Reaktionsschritt der Telomerisation kann ganz allgemein nach bekannten technischen Verfahren, insbesondere durch thermische Trennverfahren, wie beispielsweise Destillation oder Extraktion erfolgen. Es kann beispielsweise eine destillative Trennung in folgende Fraktionen erfolgen:
- eine C₄-Fraktion, die n-Butan, Isobutan, 1-Buten, 2-Butene, Isobuten, 1,3-Butadien, 1,2-Butadien und gegebenenfalls ganz oder teilweise das Nucleophil enthält,
- eine Fraktion mit dem Zielprodukt (2,7-Octadienylderivat),
- eine Fraktion mit dem Nebenprodukt und/oder
- eine Fraktion mit dem Katalysator und
- gegebenenfalls eine Fraktion mit dem Nucleophil und/oder
- gegebenenfalls eine Lösemittelfraktion

Die Fraktion mit dem Nucleophil, die Fraktion mit dem Lösemittel sowie die Fraktion mit dem Katalysator kann jeweils ganz oder teilweise, gemeinsam oder getrennt in den Reaktionsschritt zurückgeführt oder aber einer Aufarbeitung zugeführt werden. Vorzugsweise werden diese Fraktionen in den Reaktionsschritt zurückgeführt.

Die Auftrennung kann aber auch so durchgeführt werden, dass nur zwei Fraktionen erhalten werden. Die eine Fraktion enthält in diesem Fall den wesentlichen Teil des Zielproduktes und die zweite Fraktion enthält den wesentlichen Teil des eingesetzten Katalysators. Die Katalysator enthaltende Fraktion wird wiederum ganz oder teilweise in den Reaktionsschritt zurückgeführt.

Das Zielprodukt wird als solches verwandt oder dient als Vorstufe für andere Stoffe. Beispielsweise kann aus dem Zielprodukt 1-Methoxyoctadi-2,7-en durch Hydrierung der beiden Doppelbindungen und anschließende Methanol-Abspaltung 1-Octen hergestellt werden. Mittels des erfindungsgemäßen Verfahrens können also 1-Methoxyoctadi-2,7-en aufweisende Gemische hergestellt werden. Diese Gemische können dann zur Herstellung von 1-Octen verwendet werden.

Wird als Nucleophil in der Telomerisation Methanol oder Ethanol verwendet, ergibt sich bei der Verwendung von C₄-Kohlenwasserstoffströmen als Ausgangsstrom (1,3-Butadien als Ausgangsolefin) die Option, das Nucleophil nicht aus dem Reaktionsprodukt zu entfernen. Statt dessen kann der Austrag der Hydrierung des Zielprodukts (z. B. 1-Methoxyoctadi-2,7-en) direkt einer Verätherung zugeführt werden, in der der als Nucleophil eingesetzte Alkohol mit dem in dem C₄-Strom enthaltenen Isobuten zu Methyl-tert.-Butylether bzw. Ethyl-tert.-Butylether umgesetzt wird. Auch diese Umsetzung erfolgt nach in der Technik bekannten Verfahren, meist unter Katalyse von Ionentauschern. Für einen vollständigen Umsatz des Isobutens muss gegebenenfalls zusätzlicher Alkohol zugefügt werden.

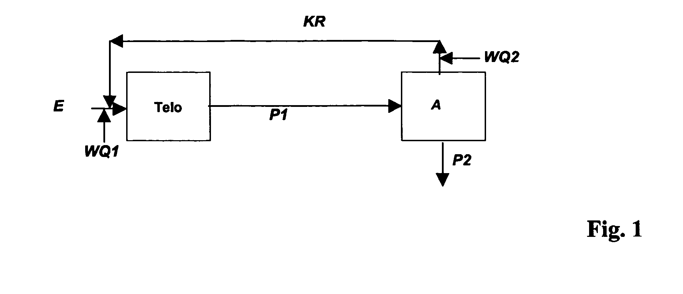

Die Erfindung wird an Hand der Figur Fig. 1 näher erläutert, ohne dass die Erfindung auf die in Fig. 1 dargestellte Ausführungsform beschränkt sein soll. In Fig.1 ist schematisch eine mögliche Ausführungsform des erfindungsgemäßen Verfahrens dargestellt. Der hier dargestellte Gesamtprozess der Telomerisation weist drei Teilschritte auf. Einen Reaktionsschritt der Telomerisation (Telo), einen Abtrennschritt (A) und einen Schritt der Katalysatorrückführung (KR). In den Reaktionsschritt (Telo) wird ein Eduktstrom gemeinsam mit dem Katalysatorrückführstrom (KR) und gegebenenfalls einer Wasserstoffquelle (WQ1) gefahren. Das als Reaktionsprodukt (P1) erhaltene Reaktionsgemisch wird in den folgenden Teilschritt (A) gefahren, in dem der Katalysator und gegebenenfalls ein Lösemittel von den übrigen Bestandteilen des Reaktionsgemisches abgetrennt wird. Das von Katalysator befreite Produktgemisch (P2) kann einer weiteren Aufarbeitung zugeführt werden. Der abgetrennte Katalysator wird über die Katalysatorrückführung (KR) dem Eduktstrom wieder zugeführt. In der Katalysatorrückführung kann ebenfalls eine Wasserstoffquelle (WQ2) dem Katalysatorstrom zugegeben werden. Bei der Durchführung des erfindungsgemäßen Verfahrens kann entweder über WQ1 oder WQ2 oder auch über WQ1 und WQ2 eine Wasserstoffquelle in einen oder mehrere Teilschritte des Gesamtprozesses der Telomerisation zugeführt werden. Es versteht sich von selbst, dass bei der Durchführung des Verfahrens evtl. weitere Vorrichtungen wie z. B. Pumpen und Ventile etc. oder Möglichkeiten z. B. zur Ausschleusung eines Teils des gebrauchten Katalysators und/oder zur Zugabe von Frischkatalysator vorhanden sein können oder müssen, die in dem Schema gemäß Fig. 1 aber nicht dargestellt sind.

Die folgenden Beispiele sollen die Erfindung näher erläutern, ohne den Schutzbereich zu beschränken, der sich aus der Beschreibung und den Patenansprüchen ergibt.

### Beispiele

### Beispiel 1: Telomerisation ohne Anwesenheit von Alkinen

In einem 100-ml-Schlenkrohr wurden unter Schutzgas 55,9 mg (0,18 mmol) Palladiumacetylacetonat und 0,390 g (0,75 mmol) 1,3-Bis(2,4,6-trimethylphenyl)imidazolium-o-kresolat-o-kresol in 51,2 g (1,59 mol) Methanol gelöst. In einem 3-Liter-Autoklaven der Firma Büchi wurden 6,72 g (0,06 mol) o-Kresol - im Wasserbad auf 40 °C erwärmt - und 3,47 g (0,06 mol) Natriummethanolat in 115 g (3,59 mol) Methanol und in 100 g (0,52 mol) Tripropylenglykol gelöst. Anschließend wurde mit Hilfe einer Gasdruckdose 536 g eines C₄-Kohlenwasserstoffgemisches in den Autoklaven hineingedrückt (Mengenbestimmung durch Massenverlust in der C₄-Vorratsflasche). Der Autoklav wurde unter Rühren auf Reaktionstemperatur (80 °C) erwärmt, die Palladium-haltige Lösung wurde in einer Portion zum Autoklaven-Inhalt gegeben und die Reaktion mit Hilfe eines online-Gaschromatographen verfolgt. Die Reaktionsdauer betrug 14 h. Als Kohlenwasserstoffgemisch wurde ein alkinfreies C₄-Kohlenwasserstoffgemisch mit 42,61 Massen% 1,3-Butadien, 1,77 Massen% i-Butan, 7,05 Massen-% n-Butan, 5,14 Massen% trans-Buten, 15,05 Massen% 1-Buten, 24,80 Massen% i-Buten, 3,58 Massen-% cis-Buten (keine Alkine nachweisbar).

### GC-Analytik:

**GC** (1. Säule: DB-WAX/A12O3, 2. Säule: DB-Wax/HP-5MS; Änfangstemperatur: 50 GC, Maximum-Temperatur: 200 °C, Anfangszeit: 1 min, Äquilibrierungszeit: 3 min; Temperaturprogramm: von 50 °C mit 15 °C min⁻¹ auf 200 °C, Laufzeit: 11 min; Inj.: 220 °C, konst. Fluss). Retentionszeit[tR](C₄-Kohlenwasserstoffe) = 2.762 min,
tR(Methanol) = 3.152 min, tR(1,7-Octadien) = 3.866 min, tR(trans-1,6-Octadien) = 3.958 min, tR(cis-1,6-Octadien) = 4.030 min, tR(cis-1,3,7-Octatrien) = 4.291 min, tR(trans-1,3,7-Octatrien) = 4.292 min, tR(Vinylcyclohexen) = 4.448 min, tR(i-Butan) = 4.552 min, tR(n-Butan) = 4.822 min, tR(3-MODE) = 5.523 min, tR(trans-Buten) = 6.116 min, tR(1-Buten) = 6.240 min, tR(i-Buten) = 6.412 min, tR(cis-Buten) = 6.616 min, tR(1-MODE) = 6.650 min, tR(1,2-Butadien) = 6.900 min, tR(1,3-Butadien) = 7.526 min.
2,7-Octadienyl-1-methylether (= 1-MODE)
1,7-Octadien-3-methylether (= 3-MODE)

### Beispiel 2: Telomerisation in Gegenwart von 2273 Massen-ppm an Acetylenen

### (Vergleichsversuch)

In einem 100 ml Schlenkrohr wurden unter Schutzgas 56,2 mg (0,18 mmol) Palladiumacetylacetonat und 0,395 g (0,76 mmol) 1,3-Bis(2,4,6-trimethylphenyl)imidazolium-o-kresolat-o-kresol in 51,2 g (1,59 mol) Methanol gelöst. In einem 3-Liter-Autoklaven der Firma Büchi wurden 6,80 g (0,062 mol) o-Kresol - im Wasserbad auf 40 °C erwärmt - und 3,80 g (0,07 mol) Natriummethanolat in 115,1 g (3,59 mol) Methanol und in 101,1 g (0,52 mol) Tripropylenglykol gelöst. Anschließend wurde mit Hilfe einer Gasdruckdose 573 g eines C4-Kohlenwasserstoffgemisches in den Autoklaven hineingedrückt (Mengenbestimmung durch Massenverlust in der C₄-Vorratsflasche). Der Autoklav wurde unter Rühren auf Reaktionstemperatur (80 °C) erwärmt, die palladiumhaltige Lösung wurde zum Autoklaven-Inhalt gegeben und die Reaktion mit Hilfe eines online Gaschromatographen verfolgt. Die Reaktionsdauer betrug 14 h

GC (1. Säule: DB-WAX/Al₂O₃, 2.Säule: DB-Wax/HP5MS; Anfangstemperatur: 50 °C, Maximum-Temperatur: 200 °C, Anfangszeit: 1 min, Equilibrierungszeit: 3 min; Temperaturprogramm: von 50 °C mit 15 °C min⁻¹ auf 200 °C, Laufzeit: 11 min; Inj.: 220 °C, konst. Fluss), tR(C4-Kohlenwasserstoffe) = 2.762 min, tR(Methanol) = 3.152 min, tR(1,7-Octadien) = 3.866 min, tR(trans-1,6-Octadien) = 3.958 min, tR(cis-1,6-Octadien) = 4.030 min, tR(cis-1,3,7-Octatrien) = 4.291 min, tR(trans-1,3,7-Octatrien) = 4.292 min, tR(Vinylcyclohexen) = 4.448 min, tR(i-Butan) = 4.552 min, tR(n-Butan) = 4.822 min, tR(3-MODE) = 5.523 min, tR(trans-Buten) = 6.116 min, tR(1-Buten) = 6.240 min, tR(i-Buten) = 6.412 min, tR(cis-Buten) = 6.616 min, tR(1-MODE) = 6.650 min, tR(1,2-Butadien) = 6.900 min, tR(1,3-Butadien) = 7.526 min.
2,7-Octadienyl-1-methylether (=1-MODE)
1,7-Octadien-3-methylether (=3-MODE)

In dem erfindungsgemäßen Beispiel wurde ein C₄-Kohlenwasserstoffgemisch mit 45,39 Massen-% 1,3-Butadien, 1,46 Massen% i-Butan, 4,61 Massen-% n-Butan, 5,20 Massen-% trans-Buten, 15,22 Massen-% 1-Buten, 23,85 Massen% cis-Buten, 0,1866 Massen-% Vinylacetylen und 0,0407 Massen-% 1-Butin eingesetzt.

### Beispiel 3: Telomerisation unter Wasserstoffzugabe (gemäß der Erfindung):

In einem 100 ml Schlenkrohr wurden unter Schutzgas 55 mg (0,181 mmol) Palladiumacetylacetonat und 0,384 g (0,96 mmol) 1,3-Bis(2,4,6-trimethylphenyl)-imidazolium-o-kresolat-o-kresol in 50,2 g (1,57 mol) Methanol gelöst. In einem 3-Liter-Autoklaven der Firma Büchi wurden 6,98 g (0,064 mol) o-Kresol - im Wasserbad auf 40 °C erwärmt - und 3,95 g (0,070 mol) Natriummethanolat in 115,2 g (3,59 mol) Methanol und in 100,7 g (0,52 mol) Tripropylenglykol gelöst. Anschließend wurde mit Hilfe einer Gasdruckdose 526 g eines C₄-Kohlenwasserstoffgemisches in den Autoklaven hineingedrückt (Mengenbestimmung durch Massenverlust in der C₄-Vorratsflasche).
Ohne das Reaktionsgemisch aufzuheizen, wurden nach 1 min, 3h 2 min, 22 h 35 min, 25 h 10 min, 27 h 11 min, 29 h 50 min, und 49 h 10 min jeweils 0,1 MPa Wasserstoff aufgedrückt und die Konzentration der Alkine in der gasförmigen C₄-Probe mittels einem Gaschromatographen (GC) gemessen. Der Autoklav wurde nach 52 h 45 min unter Rühren auf Reaktionstemperatur (80 °C) erwärmt. Die Reaktionsdauer bei 80 °C betrug 8,5 h.

GC (1. Säule: DB-WAX/Al₂O₃, 2.Säule: DB-Wax/HPSMS; Anfangstemperatur: 50 °C, Maximum-Temperatur: 200 °C, Anfangszeit: 1 min, Äquilibrierungszeit: 3 min; Temperaturprogramm: von 50 °C mit 15°C min⁻¹ auf 200 °C, Laufzeit: 11 min; Inj.: 220 °C, konst. Fluss), tR(C₄-Kohlenwasserstoffe) = 2762 min, tR(Methanol) = 3152 min, tR(1,7-Octadien) = 3866 min, tR(trans-1,6-Octadien) = 3958 min, tR(cis-1,6-Octadien) = 4030 min, tR(cis-1,3,7-Octatrien) = 4291 min, tR(trans-1,3,7-Octatrien) = 4292 min, tR(Vinylcyclohexen) = 4448 min, tR(i-Butan) = 4552 min, tR(n-Butan) 4822 min, tR(3-MODE) = 5523 min, tR(trans-Buten) = 6116 min, tR(1-Buten) = 6240 min, tR(i-Buten) = 6412 min, tR(cis-Buten) = 6616 min, tR(1-MODE) = 6650 min, tR(1,2-Butadien) = 6900 min, tR(1,3-Butadien) = 7526 min.
2,7-Octadienyl-1-methylether (=1-MODE)
1,7-Octadien-3-methylether (=3-MODE)

In dem erfindungsgemäßen Beispiel wurde ein C₄-Kohlenwasserstoffgemisch mit 43,48 Massen% 1,3-Butadien, 3,58 Massen-% i-Butan, 5,69 Massen-% n-Butan, 4,00 Massen-% trans-Buten, 14,78 Massen-% 1-Buten, 24,9 Massen% i-Buten, 2,56 Massen% cis-Buten und 0,005 Massen-% Propin, 0,6299 Massen% Vinylacetylen und 0,1058 Massen-% 1-Butin (7407 Massen-ppm an Alkinen) eingesetzt

### Beispiel 4: Telomerisatlon unter Wasserstoffzugabe (gemäß der Erfindung):

In einem 100 ml Schlenkrohr wurden unter Schutzgas 57,6 mg (0,189 mmol) Palladiumacetylacetonat und 0,399 g (0,76 mmol) 1,3-Bis(2,4,6.trimethylphenyl)imidazolium-o-kresolat-o-kresol in 51,3 g (1,60 mol) Methanol gelöst. In einem 3-Liter-Autoklaven der Firma Büchi wurden 6,90 g (0,064 mol) o-Kresol - im Wasserbad auf 40 °C erwärmt - und 3,80 g (0,070 mol) Natriummethanolat in 115,1 g (3,59 mol) Methanol und in 102 g (0,53 mol) Tripropylenglykol gelöst. Anschließend wurde mit Hilfe einer Gasdruckdose 495 g eines C₄-Kohlenwasserstoffgemisches in den Autoklaven hineingedrückt (Mengenbestimmung durch Massenverlust in der C₄-Vorratsflasche). Der Autoklav wurde unter Rühren auf Reaktionstemperatur (80 °C) erwärmt und die palladiumhaltige Lösung wurde zum Autoklaven-Inhalt gegeben. Als nächstes wurden 1,4 MPa Wasserstoff aufgedrückt und die Reaktion mit Hilfe eines online Gaschromatographen verfolgt. Die Reaktionsdauer betrug 14 h

GC (1. Säule: DB-WAX/Al₂O₃, 2.Säule: DB-Wax/HP5MS; Anfangstemperatur: 50 °C, Maximum-Temperatur: 200 °C, Anfangszeit: 1 min, Äquilibrierungszeit: 3 min; Temperaturprogramm: von 50 °C mit 15 °C min⁻¹ auf 200 °C, Laufzeit: 11 min; Inj.: 220 °C, konst. Fluss), tR(C₄-Kohlenwasserstoffe) = 2762 min, tR(Methanol) = 3152 min, tR(1,7-Octadien) = 3866 min, tR(trans-1,6-Octadien) = 3958 min, tR(cis-1,6-Octadien) = 4030 min, tR(cis-1,3,7-Octatrien) = 4291 min, tR(trans-1,3,7-Octatrien) = 4292 min, tR(Vinylcyclohexen) = 4448 min, tR(i-Butan) = 4552 min, tR(n-Butan) 4822 min, tR(3-MODE) = 5523 min, tR(trans-Buten) = 6116 min, tR(1-Buten) = 6240 min, tR(i-Buten) = 6412 min, tR(cis-Buten) = 6616 min, tR(1-MODE) = 6650 min, tR(1,2-Butadien) = 6900 min, tR(1,3-Butadien) = 7526 min.
2,7-Octadienyl-1-methylether (=1-MODE)
1,7-Octadien-3-methylether (=3-MODE)

In dem erfindungsgemäßen Beispiel wurde ein C₄-Kohlenwasserstoffgemisch mit 44,69 Massen% 1,3-Butadien, 2,56 Massen-% i-Butan, 4,82 Massen-% n-Butan, 3,96 Massen% trans-Buten, 15,50 Massen-% 1-Buten, 24,68 Massen-% iso-Buten, 2,76 Massen-% cis-Buten und 0,0415 Massen-% Propin, 0,4609 Massen% Vinylacetylen und 0,2328 Massen% 1-Butin (7350 Massen-ppm an Alkinen) eingesetzt.

**Tabelle 2: Zusanvnenfassung der Ergebnisse der Beispiele 1 bis 4**

| **Beispiel 1** | | | | **Beispiel 2** | | | | **Beispiel 3** | | | | **Beispiel 4** | | | |
|---|---|---|---|---|---|---|---|---|---|---|---|---|---|---|---|
| Zeit min | Umsatz % | Sel. MODE %* | Ausb. MODE | Zeit min | Umsatz % | Sel. MODE %* | Ausb. MODE | Zeit min | Umsatz % | Sel. MODE %* | Ausb. MODE % | Zeit Min | Umsatz % | Sel. MODE %* | Ausb. MODE |
| 0 | 0,67 | 100 | 0,5 | 0 | 2,6 | 100 | 2,6 | 0 | 3,62 | 100 | 3,2 | 0 | 2,57 | 96,80 | 2,6 |
| 140 | 65,22 | 96,53 | 62,0 | 140 | 1,53 | 90,48 | 1,4 | 140 | 84,07 | 96,59 | 75,3 | 140 | 37,45 | 96,42 | 26,0 |
| 350 | 97,71 | 96,37 | 93,3 | 380 | 1,75 | 82,18 | 1,4 | 330 | 98,0 | 96,27 | 88,3 | 350 | 96,22 | 96,83 | 74,8 |
| 710 | 99,33 | 95,89 | 94,4 | 800 | 2,18 | 72,13 | 1,6 | 510 | 98,64 | 95,82 | 89,1 | 770 | 99,06 | 95,76 | 76,1 |

| | | | | | | | | | | | | | | | |
|---|---|---|---|---|---|---|---|---|---|---|---|---|---|---|---|
| * Sel. MODE = Selektivität MODE unter C8-Produkten Ausb. % MODE = mol MODE (aus GC) pro mol Butadien im Edukt * 2* 100% | | | | | | | | | | | | | | | |

Wie durch den Vergleich der Beispiele 1 und 2 leicht zu erkennen ist, führt das Vorhandensein von Acetylenen im verwendeten C₄-Kohlenwasserstoffgenüsch zu einer Inhibierung des Katalysators, so dass eine Telomerisation unter ansonsten gleichen Bedingungen nicht stattfindet. In Beispiel 3 wird von einem C₄-Kohlenwasserstoffgemisch ausgegangen, welches einen noch größeren Anteil an Alkanen aufweist. Dem Reaktionsgemisch wird regelmäßig Wasserstoff zugegeben. Nach ca. 37 Stunden, ist ein langsamer Start der Reaktion auch bei niedriger Temperatur zu beobachten. Nach einer anschließenden Temperaturerhöhung kann trotz des Vorhandenseins von Alkinen im Ausgangs-C₄-Kohlenwasserstoffgemisch eine Telomerisation beobachtet werden. Durch die erfindungsgemäße Zugabe einer Wasserstoffquelle zu dem Reaktionsgemisch, konnte der inhibierte Katalysator wieder aktiviert werden.

Auch durch Beispiel 4 wird belegt, dass durch Gegenwart von Wasserstoff in der Telomerisierungs-Reaktion die inhibierende Wirkung der Alkine neutralisiert werden kann. In Beispiel 4 wird der Wasserstoff kurz vor dem Start der Reaktion zudosiert und die Mischung sogleich auf 80 °C erhitzt. Trotz der gegenüber Beispiel 2 höheren Alkinmenge ist in Gegenwart von Wasserstoff ein hohes Ausmaß an Produkt-Bildung zu beobachten, auch wenn die Reaktionsgeschwindigkeit gegenüber Beispiel 1 nicht voll wiederhergestellt werden konnte. Als Nebenreaktion wird eine Hydrierung von Butadien zu den Butenen beobachtet

Der im Vergleich zu den Beispielen 1 und 4 besonders hohe Umsatz zu Beginn der Reaktion in Beispiel 3 ist vermutlich darauf zurückzuführen, dass die den Katalysator desaktivierenden Alkine vor dem Beginn der eigentlichen Reaktion in Gegenwart des Telomerisationskatalysators bereits vollständig durch Hydrieren entfernt wurden und gegebenenfalls desaktivierter Katalysator wieder aktiviert wurde. Die größere Ausbeute an MODE in Beispiel 3 im Vergleich zu Beispiel 4 liegt vermutlich darin begründet, dass bei der einmaligen Zugabe von Wasserstoff gemäß Beispiel 4 ein so großer Überschuss an Wasserstoff im Reaktionsgemisch vorhanden ist, dass die Hydrierung von Butadien zu Butenen als Konkurrenzreaktion zur Hydrierung der Alkine vermehrt abläuft. Ein weiter Grund dafür, dass im Beispiel 3 die MODE-Menge bei vergleichbarer Verweilzeit/Reaktionsdauer größer ist, könnte der sein, dass die Regenerierung nicht sehr schnell abläuft, parallel aber schon die Hydrierung und Telomerisation ablaufen, so dass weniger aktiver Katalysator zur Verfügung steht. Der Vorteil der Ausfiihrungsform des erfindungsgemäßen Verfahrens gemäß Beispiel 4 liegt darin, dass auf einen zeitintensiven Schritt der Vorhydrierung bzw. Vorregenerierung, wie in Beispiel 3 durchgeführt, verzichtet werden kann.

## Patentansprüche

1. Verfahren zur Telomerisation von nicht cyclischen Olefinen mit mindestens zwei konjugierten Doppelbindungen (VI) mit mindestens einem Nucleophil (VII) unter Verwendung eines Katalysators enthaltend ein Metall der Gruppe 8, 9 oder 10 des Periodensystems der Elemente,
**dadurch gekennzeichnet,**
**dass** im Gesamtprozess der Telomerisation zumindest ein Teilschritt vorhanden ist, bei dem über eine Wasserstoffquelle, ausgewählt aus einem Wasserstoff aufweisenden Gas, dem in diesem Teilschritt vorliegenden Prozessgemisch Wasserstoff zugegeben wird.

2. Verfahren gemäß Anspruch 1,
**dadurch gekennzeichnet,**
**dass** als Einsatzstoff ein Gemisch von Kohlenwasserstoffen eingesetzt wird, das die nicht cyclischen Olefine mit mindestens zwei konjugierten Doppelbindungen in Mischung mit anderen Kohlenwasserstoffen aufweist.

3. Verfahren gemäß Anspruch 2,
**dadurch gekennzeichnet,**
**dass** das Gemisch bis zu 5 Massen-% Alkine enthält.

4. Verfahren gemäß Anspruch 3,
**dadurch gekennzeichnet,**
**dass** das Gemisch Alkine, ausgewählt aus Vinylacetylen und 1-Butin enthält.

5. Verfahren gemäß Anspruch 3 oder 4,
**dadurch gekennzeichnet,**
**dass** ein Teil der vorhandenen Alkine vor Einsatz in der Telomerisation selektiv hydriert wird.

6. Verfahren gemäß zumindest einem der Ansprüche 3 bis 5,
**dadurch gekennzeichnet,**
**dass** durch die Wasserstoffquelle soviel Wasserstoff dem Gesamtprozess zugeführt wird, dass das molare Verhältnis von Wasserstoff zu Alkin zumindest 1 zu 1 beträgt.

7. Verfahren gemäß zumindest einem der Ansprüche 2 bis 6,
**dadurch gekennzeichnet,**
**dass** das Gemisch zu über 90 Massen-% C₄-Kohlenwasserstoffe enthält.

8. Verfahren gemäß zumindest einem der Ansprüche 1 bis 7,
**dadurch gekennzeichnet,**
**dass** als nicht cyclische Olefine 1,3-Butadien oder Isopren eingesetzt wird.

9. Verfahren nach zumindest einem der Ansprüche 1 bis 8,
**dadurch gekennzeichnet,**
**dass** ein Palladiumcarbenkomplex als Telomerisationskatalysator eingesetzt wird, der einen Carbenliganden der allgemeinen Formel VIII aufweist: 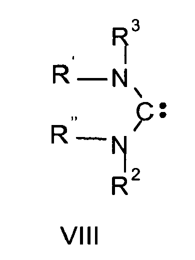 mit
R²; R³: gleich oder verschieden a) lineare, verzweigte, substituierte oder unsubstituierte cyclische oder alicyclische Alkylgruppen mit 1 bis 24 Kohlenstoffatomen, oder b) substituierte oder unsubstituierte, mono- oder polycyclische Arylgruppen mit 6 bis 24 Kohlenstoffatomen oder c) mono- oder polycyclischer, substituierter oder unsubstituierter Heterocyclus mit 4 bis 24 Kohlenstoffatomen und mindestens einem Heteroatom aus der Gruppe N, O und S und
R', R": gleich oder verschiedenen Wasserstoff, Alkyl, Aryl, Heteroaryl, -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, -PO₃H₂, wobei die Alkylgruppen 1 - 24 und die Aryl- und Heteroarylgruppen 5 bis 24 Kohlenstoffatome beinhalten und die Reste R' und R" auch Teil eines verbrückenden aliphatischen oder aromatischen Rings sein können.

10. Verfahren nach zumindest einem der Ansprüche 1 bis 8,
**dadurch gekennzeichnet,**
**dass** ein Palladiumcarbenkomplex als Telomerisationskatalysator eingesetzt wird, der einen Carbenliganden der allgemeinen Formel IX, X, XI oder XII aufweist: 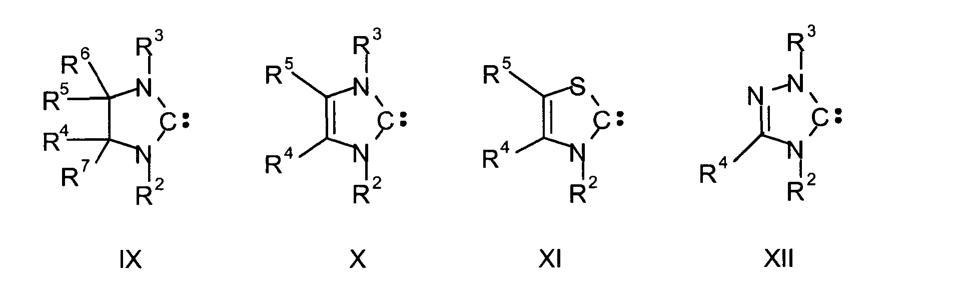 mit
R²; R³: gleich oder verschieden a) lineare, verzweigte, substituierte oder unsubstituierte cyclische oder alicyclische Alkylgruppen mit 1 bis 24 Kohlenstoffatomen, oder b) substituierte oder unsubstituierte, mono- oder polycyclische Arylgruppen mit 6 bis 24 Kohlenstoffatomen oder c) mono- oder polycyclischer, substituierter oder unsubstituierter Heterocyclus mit 4 bis 24 Kohlenstoffatomen und mindestens einem Heteroatom aus der Gruppe N, O und S, und
R⁴, R⁵, R⁶, R⁷: gleich oder verschiedenen Wasserstoff, Alkyl, Aryl, Heteroaryl, -CN, -COOH, -COO-Alkyl-, -COO-Aryl-, -OCO-Alkyl-, -OCO-Aryl-, -OCOO-Allcyl-, -OCOO-Aryl-, -CHO, -CO-Alkyl-, -CO-Aryl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, -PO₃H₂, wobei die Alkylgruppen 1 - 24 und die Aryl- und Heteroarylgruppen 5 bis 24 Kohlenstoffatome beinhalten und die Reste R⁴, R⁵, R⁶ und R⁷ auch Teil eines verbrückenden aliphatischen oder aromatischen Rings sein können.

11. Verfahren nach einem der Ansprüche 9 oder 10,
**dadurch gekennzeichnet,**
**dass** das Verhältnis von Ligand zu Metall [Mol/Mol] in dem Reaktionsgemisch 0,01 zu 1 bis 250 zu 1 beträgt.

12. Verfahren nach einem der Ansprüche 1 bis 11,
**dadurch gekennzeichnet,**
**dass** als Nucleophil VII Verbindungen der allgemeinen Formeln VIIa, VIIb oder VIIc
R¹-O-H VIIa
(R^{1a})(R ^{1b})N-H VIIb
R^{1a}-COOH VIIc
worin R^{1a} und R^{1b} unabhängig voneinander ausgewählt sind aus Wasserstoff, einer linearen, verzweigten oder cyclischen C₁ bis C₂₂-Alkylgruppe, -Alkenylgruppe oder -Alkinylgruppe, einer C₅ bis C₁₈-Arylgruppe oder einer -CO-Alkyl-(C₁-C₈)-Gruppe oder einer -CO-Aryl-(C₅-C₁₀)-Gruppe, wobei diese Gruppen Substituenten ausgewählt aus der Gruppe -CN, -COOH, -COO-Alkyl-(C₁-C₈), -CO-Alkyl-(C₁-C₈), -Aryl-(C₅-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃ und -NO₂ enthalten können, und wobei die Reste R^{1a} und R^{1b} über kovalente Bindungen miteinander verknüpft sein können, eingesetzt werden.

13. Verfahren nach Anspruch 12,
**dadurch gekennzeichnet,**
**dass** als Nucleophil (VII) Wasser, Alkohole, Phenole, Polyole, Carbonsäuren, Ammoniak und/oder primäre oder sekundäre Amine eingesetzt werden.

14. Verfahren nach einem der Ansprüche 1 bis 13,
**dadurch gekennzeichnet,**
**dass** die Telomerisation in Anwesenheit eines Lösemittels durchgeführt wird.

15. Verfahren nach einem der Ansprüche 1 bis 14,
**dadurch gekennzeichnet,**
**dass** die Telomerisation in Anwesenheit eines Lösemittels durchgeführt wird, wobei als Lösemittel das Nucleophil (VII) und/oder inerte organische Lösemittel eingesetzt werden.

16. Verfahren nach einem der Ansprüche 1 bis 15,
**dadurch gekennzeichnet,**
**dass** die Wasserstoffquelle dem Gesamtprozess der Telomerisation im Reaktionsschritt zugegeben wird.

17. Verfahren nach einem der Ansprüche 1 bis 16,
**dadurch gekennzeichnet,**
**dass** der Gesamtprozess der Telomerisation einen Schritt der Katalysatorrückfiihrung aufweist und die Wasserstoffquelle dem Gesamtprozess der Telomerisation im Schritt der Katalysatorrückfiihrung zugegeben wird.

## Claims

1. Process for telomerizing noncyclic olefins having at least two conjugated double bonds (VI) with at least one nucleophile (VII) using a catalyst containing a metal of group 8, 9 or 10 of the Periodic Table of the Elements,
**characterized in that**
the overall telomerization process includes at least one step in which hydrogen is added via a hydrogen source selected from a gas comprising hydrogen to the process mixture present in this step.

2. Process according to Claim 1,
**characterized in that**
the feedstock used is a mixture of hydrocarbons which comprises the noncyclic olefins having at least two conjugated double bonds in a mixture with other hydrocarbons.

3. Process according to Claim 2,
**characterized in that**
the mixture contains up to 5% by mass of alkynes.

4. Process according to Claim 3,
**characterized in that**
the mixture comprises alkynes selected from vinylacetylene and 1-butyne.

5. Process according to Claim 3 or 4,
**characterized in that**
a portion of the alkynes present is hydrogenated selectively before use in the telomerization.

6. Process according to at least one of Claims 3 to 5, **characterized in that**
the hydrogen source feeds sufficient hydrogen to the overall process that the molar ratio of hydrogen to alkyne is at least 1 to 1.

7. Process according to at least one of Claims 2 to 6, **characterized in that**
the mixture contains over 90% by mass of C₄ hydrocarbons.

8. Process according to at least one of Claims 1 to 7, **characterized in that**
the noncyclic olefins used are 1,3-butadiene or isoprene.

9. Process according to at least one of Claims 1 to 8, **characterized in that**
the telomerization catalyst used is a palladium-carbene complex which contains a carbene ligand of the general formula VIII: 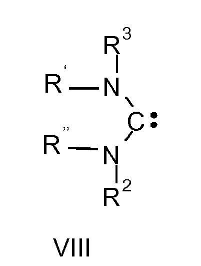 where
R²; R³: are the same or different and are each a) linear, branched, substituted or unsubstituted, cyclic or alicyclic alkyl groups having from 1 to 24 carbon atoms, or b) substituted or unsubstituted, mono- or polycyclic aryl groups having from 6 to 24 carbon atoms or c) mono- or polycyclic, substituted or unsubstituted heterocycle having from 4 to 24 carbon atoms and at least one heteroatom from the group of N, 0 and S, and
R', R": are the same or different and are each hydrogen, alkyl, aryl, heteroaryl, -CN, -COOH, -COO-alkyl-, -COO-aryl-, -OCO-alkyl-, -OCO-aryl-, -OCOO-alkyl-, -OCOO-aryl-, -CHO, -CO-alkyl-, -CO- aryl-, -0-alkyl-, -O-aryl-, -NH₂, -NH (alkyl) -, -N(alkyl)₂-, -NH(aryl)-, -N(alkyl)₂-, -F, -Cl, -Br, -I, -OH, -CF₃, -NO₂, -ferrocenyl, -SO₃H, -PO₃H₂, where the alkyl groups contain 1-24 carbon atoms and the aryl and heteroaryl groups from 5 to 24 carbon atoms, and the R' and R" radicals may also be part of a bridging aliphatic or aromatic ring.

10. Process according to at least one of Claims 1 to 8, **characterized in that**
the telomerization catalyst used is a palladium-carbene complex which contains a carbene ligand of the general formula IX, X, XI or XII: 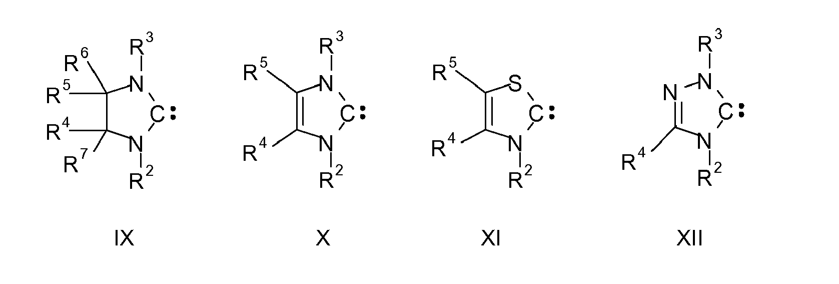 where
R²; R³: are the same or different and are each a) linear, branched, substituted or unsubstituted, cyclic or alicyclic alkyl groups having from 1 to 24 carbon atoms, or b) substituted or unsubstituted, mono- or polycyclic aryl groups having from 6 to 24 carbon atoms or c) mono- or polycyclic, substituted or unsubstituted heterocycle having from 4 to 24 carbon atoms and at least one heteroatom from the group of N, 0 and S, and
R⁴, R⁵, R⁶, R⁷: are the same or different and are each hydrogen, alkyl, aryl, heteroaryl, -CN, -COOH, -COO-alkyl-, -COO-aryl-, -OCO-alkyl-, -OCO-aryl-, -OCOO- alkyl-, -OCOO-aryl-, -CHO, -CO- alkyl-, -CO-aryl-, -O-alkyl-, -O- aryl-, -NH₂, -NH (alkyl) -, -N(alkyl)₂-, -NH(aryl)-, -N(alkyl)₂-, -F, -Cl, -Br, -I, -OH, -CF₃, -NO₂, -ferrocenyl, -SO₃H, -PO₃H₂, where the alkyl groups contain 1-24 carbon atoms and the aryl and heteroaryl groups from 5 to 24 carbon atoms, and the R⁴, R⁵, R⁶ and R⁷ radicals may also be part of a bridging aliphatic or aromatic ring.

11. Process according to one of Claims 9 or 10, **characterized in that**
the ratio of ligand to metal [mol/mol] in the reaction mixture is from 0.01:1 to 250:1.

12. Process according to one of Claims 1 to 11, **characterized in that**
the nucleophiles VII used are compounds of the general formulae VIIa, VIIb or VIIc
R^{1a}-O-H VIIa
(R^{1a})(R^{1b})N-H VIIb
R^{1a}-COOH VIIc
in which R^{1a} and R^{1b} are each independently selected from hydrogen, a linear, branched or cyclic C₁ to C₂₂-alkyl group, -alkenyl group or -alkynyl group, a C₅ to C₁₈-aryl group or a -CO-alkyl-(C₁-C₈) group or a -CO-aryl-(C₅-C₁₀₎ group, where these groups may contain substituents selected from the group of -CN, -COOH, -COO-alkyl-(C₁-C₈), -CO-alkyl- (C₁-C₈), -aryl-(C₅-C₁₀), -COO-aryl-(C₆-C₁₀), -CO-aryl-(C₆-C₁₀), -O-alkyl-(C₁-C₈), -O-CO-alkyl-(C₁-C₈), -N-alkyl₂-(C₁-C₈), -CHO, -SO₃H, -NH₂, -F, -Cl, -OH, -CF₃ and -NO₂, and where the R^{1a} and R^{1b} radicals may be joined together via covalent bonds.

13. Process according to Claim 12,
**characterized in that**
the nucleophile (VII) used is water, alcohols, phenols, polyols, carboxylic acids, ammonia and/or primary or secondary amines.

14. Process according to one of Claims 1 to 13,
**characterized in that**
the telomerization is carried out in the presence of a solvent.

15. Process according to one of Claims 1 to 14,
**characterized in that**
the telomerization is carried out in the presence of a solvent and the solvent used is the nucleophile (VII) and/or inert organic solvent.

16. Process according to one of Claims 1 to 15,
**characterized in that**
the hydrogen source is added in the reaction step to the overall telomerization process.

17. Process according to one of Claims 1 to 16,
**characterized in that**
the overall telomerization process has a catalyst recycling step and the hydrogen source is added to the overall telomerization process in the catalyst recycling step.

## Revendications

1. Procédé de télomérisation d'oléfines non cycliques présentant au moins deux doubles liaisons conjuguées (VI) avec au moins un nucléophile (VII), avec utilisation d'un catalyseur contenant un métal des groupes 8, 9 ou 10 du système périodique des éléments, **caractérisé en ce que** dans le procédé global de la télomérisation, il existe au moins une étape partielle dans laquelle de l'hydrogène est ajouté, via une source d'hydrogène choisie parmi un gaz présentant de l'hydrogène, au mélange de procédé se trouvant dans cette étape partielle.

2. Procédé selon la revendication 1, **caractérisé en ce qu'**on utilise comme substance de départ un mélange d'hydrocarbures, qui présente les oléfines non cycliques présentant au moins deux doubles liaisons conjuguées en mélange avec d'autres hydrocarbures.

3. Procédé selon la revendication 2, **caractérisé en ce que** le mélange contient jusqu'à 5% en masse d'alcynes.

4. Procédé selon la revendication 3, **caractérisé en ce que** le mélange contient des alcynes choisis parmi le vinylacétylène et le 1-butyne.

5. Procédé selon la revendication 3 ou 4, **caractérisé en ce qu'**une partie des alcynes présents est hydrogénée sélectivement avant l'utilisation dans la télomérisation.

6. Procédé selon au moins l'une quelconque des revendications 3 à 5, **caractérisé en ce que** la quantité d'hydrogène introduite dans le procédé global via la source d'hydrogène est telle que le rapport molaire d'hydrogène à alcyne est d'au moins 1:1.

7. Procédé selon au moins l'une quelconque des revendications 2 à 6, **caractérisé en ce que** le mélange contient jusqu'à plus de 90% en masse d'hydrocarbures en C₄.

8. Procédé selon au moins l'une quelconque des revendications 1 à 7, **caractérisé en ce que** du 1,3-butadiène ou de l'isoprène est utilisé comme oléfines non cycliques.

9. Procédé selon au moins l'une quelconque des revendications 1 à 8, **caractérisé en ce qu'**on utilise un complexe palladium-carbène comme catalyseur de télomérisation, qui présente un ligand de type carbène de la formule générale VIII : 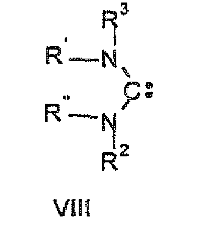 où
R² ; R³ : sont identiques ou différents et représentent
a) des groupes alkyle linéaires, ramifiés, substitués ou non substitués, cycliques ou alicycliques comprenant 1 à 24 atomes de carbone, ou
b) des groupes aryle substitués ou non substitués, monocycliques ou polycycliques comprenant 6 à 24 atomes de carbone, ou
c) des hétérocycles monocycliques ou polycycliques, substitués ou non substitués, comprenant 4 à 24 atomes de carbone et au moins un hétéroatome du groupe N, 0 et S et
R', R" : sont identiques ou différents et représentent hydrogène, alkyle, aryle, hétéroaryle, -CN, -COOH, -COO-alkyl-, -COO-aryl-, -OCO-alkyl-, -OCO-aryl-, -OCOO-alkyl-, -OCOO-aryl-, -CHO, -CO-alkyl-, -CO-aryl-, -O-alkyl-, -O-aryl-, -NH₂, -NH(alkyl)-, -N(alkyl)₂-, -NH(aryl)-, -N(alkyl)₂-, -F, -Cl, -Br, -I, -OH, -CF₃, -NO₂, -ferrocényle, -SO₃H, -PO₃H₂, où les groupes alkyle peuvent comporter 1-24 atomes de carbone et les groupes aryle et hétéroaryle 5 à 24 atomes de carbone et les radicaux R' et R" peuvent également être une partie d'un cycle aliphatique ou aromatique formant un pont.

10. Procédé selon au moins l'une quelconque des revendications 1 à 8, **caractérisé en ce qu'**on utilise un complexe palladium-carbène comme catalyseur de télomérisation, qui présente un ligand de type carbène de la formule générale IX, X, XI ou XII : 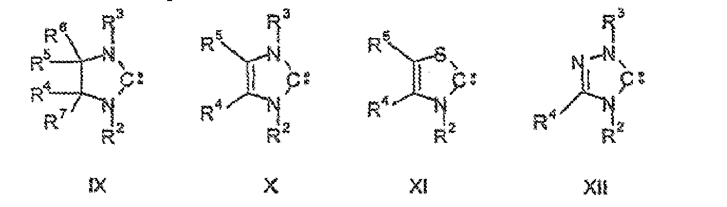 où
R² ; R³ : sont identiques ou différents et représentent
a) des groupes alkyle linéaires, ramifiés, substitués ou non substitués, cycliques ou alicycliques comprenant 1 à 24 atomes de carbone, ou
b) des groupes aryle substitués ou non substitués, monocycliques ou polycycliques comprenant 6 à 24 atomes de carbone, ou
c) des hétérocycles monocycliques ou polycycliques, substitués ou non substitués, comprenant 4 à 24 atomes de carbone et au moins un hétéroatome du groupe N, 0 et S et
R⁴, R⁵, R⁶, R⁷ : sont identiques ou différents et représentent hydrogène, alkyle, aryle, hétéroaryle, -CN, -COOH, -COO-alkyl-, -COO-aryl-, -OCO-alkyl-, -OCO-aryl-, -OCOO-alkyl-, -OCOO-aryl-, -CHO, -CO-alkyl-, -CO-aryl-, -O-alkyl-, -O-aryl-, -NH₂, -NH(alkyl)-, -N(alkyl)₂-, -NH(aryl)-, -N(alkyl)₂-, -F, -Cl, -Br, -I, -OH, -CF₃, -NO₂, -ferrocényle, -SO₃H, -PO₃H₂, où les groupes alkyle peuvent comporter 1-24 atomes de carbone et les groupes aryle et hétéroaryle 5 à 24 atomes de carbone et les radicaux R⁴, R⁵, R⁶ et R⁷ peuvent également être une partie d'un cycle aliphatique ou aromatique formant un pont.

11. Procédé selon l'une quelconque des revendications 9 ou 10, **caractérisé en ce que** le rapport de ligand à métal (mole/mole) dans le mélange réactionnel vaut 0,01:1 à 250:1.

12. Procédé selon l'une quelconque des revendications 1 à 11, **caractérisé en ce qu'**on utilise comme nucléophile VII des composés des formules générales VIIa, VIIb ou VIIc
R^{1a}-O-H VIIa
(R^{1a})(R^{1b})N-H VIIb
R^{1a}-COOH VIIc
où R^{1a} et R^{1b} sont choisis indépendamment l'un de l'autre parmi hydrogène, un groupe C₁-C₂₂-alkyle, C₁-C₂₂-alcényle ou C₁-C₂₂-alcynyle linéaire, ramifié ou cyclique, un groupe C₅-C₁₈-aryle ou un groupe -CO-(alkyle en C₁-C₈) ou un groupe -CO-(aryle en C₅-C₁₀), où ces groupes peuvent contenir des substituants choisis dans le groupe formé par -CN, -COOH, -COO-(alkyle en C₁-C₈), -CO-(alkyle en C₁-C₈), -(aryle en C₅-C₁₀), -COO-(aryle en C₆-C₁₀), -CO-(aryle en C₆-C₁₀), -O-(alkyle en C₁-C₈), -O-CO-(alkyle en C₁-C₈) , -N-(alkyle en C₁-C₈)₂, -CHO, -SO₃H, -NH₂, -F, -Cl, -OH, -CF₃ et -NO₂, et où les radicaux R^{1a} et R^{1b} peuvent être liés l'un à l'autre via des liaisons covalentes.

13. Procédé selon la revendication 12, **caractérisé en ce qu'**on utilise comme nucléophile (VII) de l'eau, des alcools, des phénols, des polyols, des acides carboxyliques, de l'ammoniac et/ou des amines primaires ou secondaires.

14. Procédé selon l'une quelconque des revendications 1 à 13, **caractérisé en ce que** la télomérisation est réalisée en présence d'un solvant.

15. Procédé selon l'une quelconque des revendications 1 à 14, **caractérisé en ce que** la télomérisation est réalisée en présence d'un solvant, où on utilise comme solvant le nucléophile (VII) et/ou des solvants organiques inertes.

16. Procédé selon l'une quelconque des revendications 1 à 15, **caractérisé en ce que** la source d'hydrogène est ajoutée au processus global de la télomérisation dans l'étape de réaction.

17. Procédé selon l'une quelconque des revendications 1 à 16, **caractérisé en ce que** le procédé global de la télomérisation présente une étape de recyclage de catalyseur et la source d'hydrogène est ajoutée au procédé global de la télomérisation dans l'étape de recyclage du catalyseur.
